# EUROPEAN PATENT SPECIFICATION

(11) **EP 2 155 669 B1**
(45) Date of publication and mention of the grant of the patent: **04.05.2011**
(21) Application number: 08760493.0
(22) Date of filing: 04.06.2008
(51) Int. Cl.: C07D 207/08, A61K 31/40, A61P 25/00

(54) **NEW DISUBSTITUTED PHENYLPYRROLIDINES AS MODULATORS OF CORTICAL CATECHOLAMINERGIC NEUROTRANSMISSION**
NEUE DISUBSTITUIERTE PHENYLPYRROLIDINE ALS MODULATOREN DER KORTIKALEN KATECHOLAMINERGEN NEUROTRANSMISSION
NOUVELLES PHÉNYLPYRROLIDINES DISUBSTITUÉES COMME MODULATEURS DE LA NEUROTRANSMISSION CATÉCHOLAMINERGIQUE CORTICALE

(30) Priority: 05.06.2007 SE 0701386; 05.06.2007 US 941993 P
(43) Date of publication of application: 24.02.2010
(73) Proprietor: NSAB, Filial af NeuroSearch Sweden AB, Sverige, 2750 Ballerup (DK)
(72) Inventor: SONESSON, Clas, S-413 46 Göteborg (SE); SWANSON, Lars, S-413 46 Göteborg (SE); PETTERSSON, Fredrik, S-413 46 Göteborg (SE); WATERS, Nicholas, S-413 46 Göteborg (SE); WATERS, Susanna, S-413 46 Göteborg (SE)
(74) Representative: Madsen, Inger Margrethe Schelde
(86) International application number: PCT/EP2008/056912
(87) International publication number: WO 2008/148799

(56) References cited:
- EP-A- 1 669 350
- WO-A-01/46146
- WO-A-92/18475
- SONESSON C ET AL: "Regioselective synthesis of 3-aryl substituted pyrrolidines via palladium catalyzed arylation: pharmacological evaluation for central dopaminergic and serotonergic activity" BIOORGANIC & MEDICINAL CHEMISTRY LETTERS, OXFORD, GB, vol. 7, no. 3, 4 February 1997 (1997-02-04), pages 241-246, XP004136001 ISSN: 0960-894X cited in the application
- SONESSON C ET AL: "SUBSTITUTED (S)-PHENYLPIPERIDINES AND RIGID CONGENERS AS PREFERENTIAL DOPAMINE AUTORECEPTOR ANTAGONISTS: SYNTHESIS AND STRUCTURE-ACTIVITY RELATIONSHIPS" JOURNAL OF MEDICINAL CHEMISTRY, USAMERICAN CHEMICAL SOCIETY. WASHINGTON, vol. 37, no. 17, 1 January 1994 (1994-01-01), pages 2735-2753, XP000925302 ISSN: 0022-2623 cited in the application

## Description

### Field of the invention

The present invention relates to new disubstituted phenylpyrrolidines and the use of these compounds which increase extracellular levels of catecholamines, dopamine and norepinephrine, in cerebral cortical areas of the mammalian brain, and more specifically to the use of 3-(disubstituted aryl)-pyrrolidines for the treatment of central nervous system disorders.

### Background of the invention

The cerebral cortex encompasses several major regions that are involved in higher functions such as thought, feelings, memory and planning (Principles of Neural science, 2nd Edition, Elsevier Science Publishing co., Inc. 1985, pp 671 - 687). Biogenic amines, i.e. dopamine, norepinephrine and serotonin, are important for mammalian cortical function. The ascending dopamine and norepinephrine pathways innervate the cortex. The serotonergic neurons of the CNS project to virtually all regions of the brain including the cerebral cortex (Fundamental Neuroscience, Academic press 1999, pp 207-212). Primary or secondary dysfunctions in the activity of these pathways lead to dysregulation of the activity at dopamine and norepinephrine and serotonin receptors in these brain areas and subsequently to manifestations of psychiatric and neurological symptoms.

The biogenic amines of the cortex modulate several aspects of cortical functions controlling affect, anxiety, motivation, cognition, attention, arousal and wakefulness (Neuropsychopharmacology, 5th generation of Progress, Lippincott, Williams and Wilkins 2002, Chapter 34). Thus, the catecholamines dopamine and norepinephrine exert strong influence on the prefrontal cortical areas, the integrity of which is essential for the so-called executive cognitive functions, related to e.g. attention, planning of actions and impulse control (the role of the catecholamines in these respects is reviewed in Arnsten and Li, 2005, Biol Psychiatry;57;1377-1384). Norepinephrine is a major part in the circuitry regulating anxiety and fear and is thus believed to be dysregulated in anxiety disorders such as panic disorders, generalized anxiety disorder (GAD) and specific phobias (Sullivan et al. 1999, Biol Psychiatry;46:1205-121). Concerning mood and affective functions, the usefulness of compounds facilitating particularly norepinephrine and serotonin neurotransmission in the treatment of depression and anxiety has strongly contributed to the widely-accepted concept that these neurotransmitters are both involved in the regulation of affective functions (Goodman & Gilman's The Pharmacological Basis of Therapeutics, Tenth Edition, McGraw-Hill, 2001).

In general, compounds specifically affecting the transmission of biogenic amines, more precisely monoamines, norepinephrine, dopamine and serotonin are successfully used to alleviate the affective, cognitive, or attentional symptoms in patients suffering from e.g. depression, anxiety and attention deficit hyperactivity disorders (ADHD).

Furthermore, the monoamine systems in the cortex are known to be directly or indirectly involved in the core symptoms of schizophrenia. Based on a synthesis of biochemical and genetic findings along with neuropsychological observations indicating dysfunction of specific cortical areas in schizophrenia, it has been proposed that this disorder emerges as various pathological etiologies converge upon cortical function leading to dysregulation of the cortical micro-circuitry, which is clinically manifested as the symptoms of schizophrenia (Harrison and Weinberger, 2005, Molecular Psychiatry; 10:40-68). This cortical micro-circuitry is regulated by several neurotransmitters, including glutamate, GABA, and dopamine.

### Description of Prior Art

Compounds belonging to the class of substituted 3-phenyl-pyrrolidiness have been reported previously. Among these compounds, some are inactive in the CNS, some display serotonergic or mixed serotonergic/dopaminergic pharmacological profiles while some are full or partial dopamine receptor agonists or antagonists with high affinity for dopamine receptors.

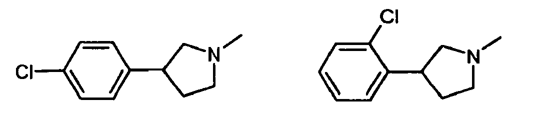

The above compounds have been disclosed as synthesis intermediates in WO 00/05225 (Preparation of biphenyl derivatives as serotonin antagonists) and by Haglid et al. as Nicotine analogs (Acta Chemica Scandinavica, 1963, 17 (6), 1743-50).

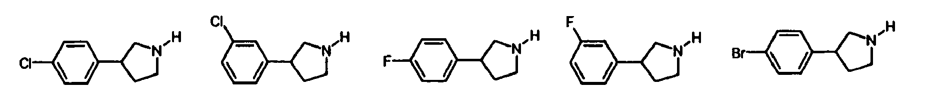

3-Chloro-phenyl-3-pyrrolidine (above) has been disclosed as synthesis intermediate in WO 2006/117669 (Preparation of hydroxyarylcarboxamide derivatives for treating cancer) and WO 2006/112685 (Preparation of triazoles and tetrazoles containing carbamoyl group as anticonvulsants). 4-Chloro-phenyl-3-pyrrolidine has been disclosed in J. Med. Chem. (2002), 45(17) 3721-3738 (Highly Potent Geminal Bisphosphonates). From Pamidronate Disodium (Aredia) to Zoledronic Acid (Zometa), Bioorganic & Medicinal Chemistry Letters (1999), 9(10), 1379-1384 (N-Substituted 3-aryipyrrolidines: potent and selective ligands at the serotonin 1A receptor), and Journal of Medicinal Chemistry (1989), 32(6) (Metabolism of 3-(p-chlorophenyl)pyrrolidine). Structural effects in conversion of a prototype γ-aminobutyric acid prodrug to lactam and γ-aminobutyric acid type metabolites). 3-Fluoro-phenyl-3-pyrrolidine has been disclosed in US 5,128,362 and EP 325963 (Preparation of 1-aminomethyl-1 ,2,3,4-tetrahydronaphthalenes as adrenergic α 2 antagonists). 4-Fluoro-phenyl-3-pyrrolidine has been disclosed in WO 2006/117669 (Preparation of hydroxyarylcarboxamide derivatives for treating cancer), Bioorganic & Medicinal Chemistry Letters (1999), 9(10), 1379-1384 (N-Substituted 3-arylpyrrolidines: potent and selective ligands at the serotonin 1A receptor), and US 5,128,362 (Preparation of 1-aminomethyl-1,2,3,4-tetrahydro naphthalenes as adrenergic α 2 antagonists). 4-Bromo-phenyl-3-pyrrolidine has been disclosed in WO 2006/117669 (Preparation of hydroxyarylcarboxamide derivatives for treating cancer), Bioorganic & Medicinal Chemistry Letters (1999), 9(10), 1379-1384 (N-Substituted 3-arylpyrrolidines: potent and selective ligands at the serotonin 1A receptor), US 5,128,362 (Preparation of 1-aminomethyl-1,2,3,4-tetrahydronaphthalenes as adrenergic α 2 antagonists), and WO 01/16136 (Preparation of tricyclic inhibitors of poly(ADP-ribose) polymerases).

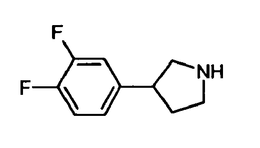

The above compound has been disclosed as a synthesis intermediate in WO 2005/028438 (Preparation of piperidine compounds as histamine H3 antagonists or inverse agonists)

Compounds with Formula 1 (WO 92/18475) have been disclosed as possessing dopaminergic stabilizer properties.

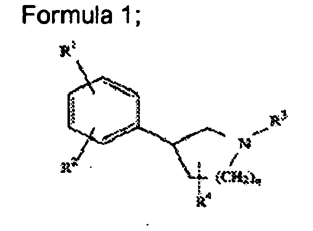

From compounds with Formula 1, Sonesson et al. (J. Med. Chem. 1994, 37, 2735-2753) have published a series of phenyl piperidines with preferential autoreceptor antagonists. The authors found the compounds to increase the DOPAC levels in striatum at 100 µmol/kg, which is a hallmark of dopamine antagonist properties. Some examples from this publication are shown: 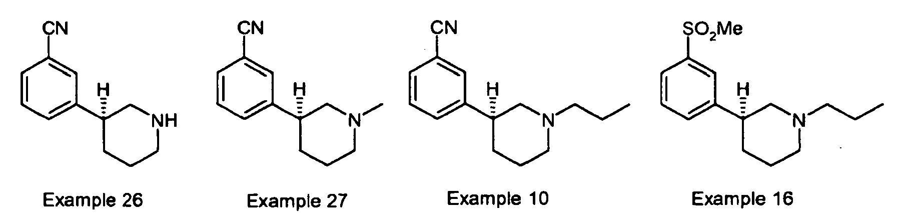 Examples from J. Med. Chem. 1994, 37, 2735 - 2753

In addition, Sonesson et al. (Bioorg. Med. Chem. Lett. 1997, 7, 241-246) have described that 3-phenyl-pyrrolidines substituted with electron withdrawing groups in the meta- position of the phenyl ring displays preferential dopamine autoreceptor antagonist properties. One example from this series is presented:

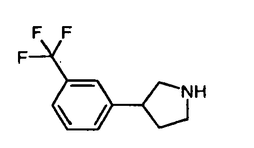

The prior art teaches that 3-phenyl-piperidines and 3-phenyl-pyrrolidines of J. Med. Chem. 1994, 37, 2735 or Bioorg. Med. Chem. Lett. 1997, 7, 241-246 have a specific, efficacious, and characteristic effect on the metabolism of dopamine, measured as increases in tissue content of DOPAC (3,4-dihydroxyphenylacetic acid) in the striatum (see Table 1). This effect on subcortical dopamine metabolism is not the objective of the present invention.

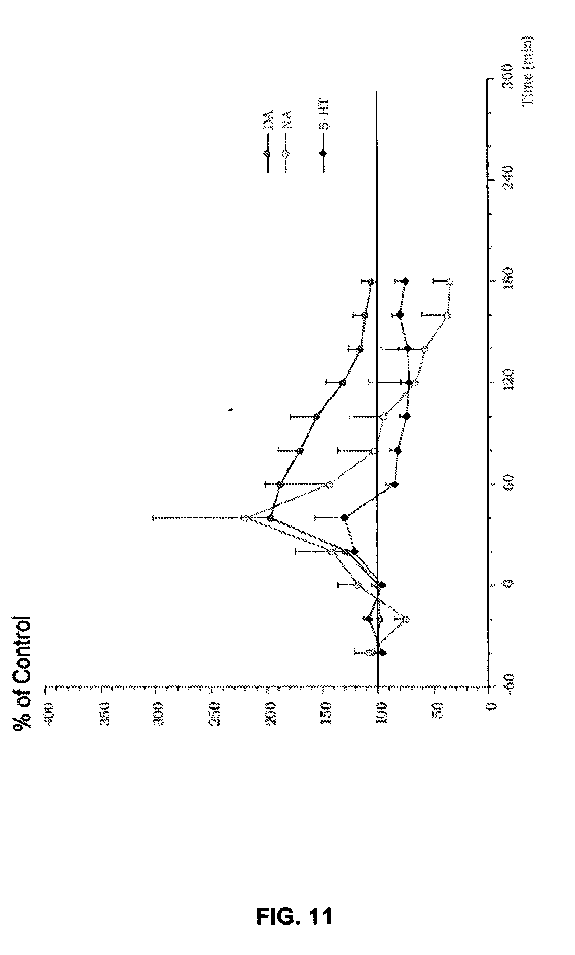

In addition, using a microdialysis technique it is shown that compounds from J. Med. Chem. 1994, 37, 2735 - 2753 were found to increase extracellular levels of monoamines, (dopamine, norepinephrine and serotonin), with equal effects in both striatum and in cerebral cortical areas of the mammalian brain (See Figures 11 - 12). In other words, the regionally selective properties of compounds of the present invention between striatum and in cerebral cortical areas are not present in the prior art.

WO 01/46146 discloses compounds with dopaminergic stabiliser properties, some of which are presented in Table 1.

Thus, there is no guidance in WO 01/46146, WO 92/18475. J. Med. Chem. 1994, 37, 2735 or Bioorg. Med. Chem. Lett. 1997, 7, 241-246, on how to obtain compounds that increase norepinephrine and dopamine neurotransmission with a preference for the frontal cortex.

### Summary of the invention

One object of the present invention is to provide new compounds for therapeutic use, and more precisely compounds with modulation of dopamine and norepinephrine neurotransmission in the mammalian brain, including the human brain. Another object of the invention is to provide compounds with therapeutic effects after oral administration. A still further object is the provision of compounds with more optimal pharmacodynamic properties such as e.g. kinetic behaviour, bioavailability, solubility or efficacy.

The present invention concerns the unexpected discovery of the pharmacological effects of compounds of the invention on monoamines in the cerebral cortex, and the use of compounds of the invention as treatment for certain CNS disorders. By pharmacological testing *in vivo* in the rat it is demonstrated that compounds of the present invention produce regionally selective increases in catecholamine levels in the frontal cortex. Due to the specific modulatory effects of the catecholamines on cortical functions related to cognition, attention and affect, the compounds of the invention can be used in the treatment of disorders characterised by dysfunctions in these areas. Thus, the compounds can be used in the treatment of cognitive disorders, ADHD, depression, and anxiety. The compounds can also be used to treat schizophrenia, which is characterised by dysfunctions of the cerebral cortex manifested in cognitive failure and psychosis.

### Detailed Description of the Invention

The following abbreviations will be used in the present invention:
NA: norepinephrine, NM: normetanephrine; DA: dopamine, DOPAC: 3,4-dihydroxyphenylacetic acid; 3-MT: 3-methoxytyramine; 5-HT: serotonin (5-hydroxytryptamine).

The present invention relates to new 3-(disubstituted aryl)-pyrrolidines, in particular 4-(*ortho,para*-disubstituted phenyl)-1- pyrrolidines, 4-(*meta,para*-disubstituted phenyl)-1-pyrrolidines, 4-(*meta,meta*-disubstituted phenyl)-1- pyrrolidines and 4-(*ortho,meta-*disubstituted phenyl)-1- pyrrolidines in the form.of the free base or pharmaceutically acceptable salts thereof, pharmaceutical compositions containing said compounds and the use of said compounds in therapy.

In its first aspect, the invention relates to a compound of Formula 2: 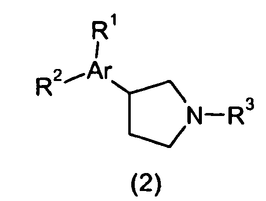 wherein:
Ar is phenyl;
R¹ is selected from the group consisting of F and Cl;
R² is selected from the group consisting of F and Cl; and
R³ is selected from the group consisting of H and Me,
with the proviso that, when Ar is phenyl and one of R¹ and R² is located in the para-position and the other of R¹ and R² is located in the meta-position, then R¹ and R² are not both F when R³ is H;
any of its stereoisomers or any mixture of its stereoisomers,
or an N-oxide thereof, or a pharmaceutically acceptable salt thereof.

In a further embodiment, the compound of the invention is a compound of Formula (3): 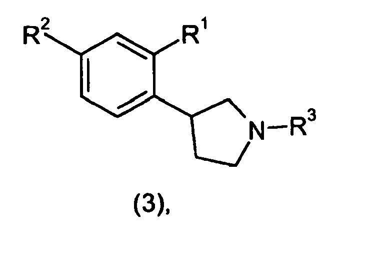 or Formula (4): 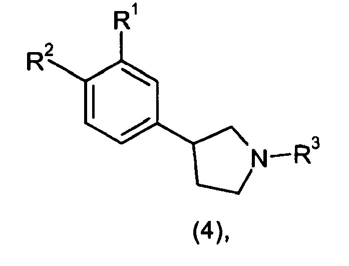 or Formula (5): 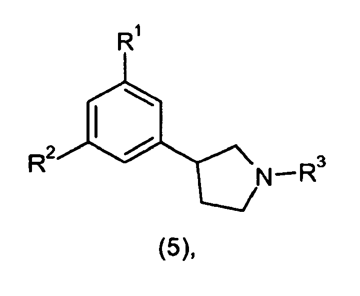 or Formula (6): 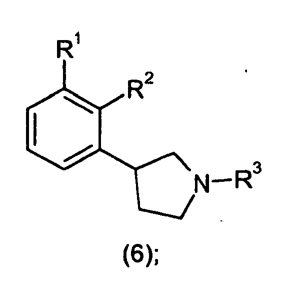 wherein R¹, R² and R³ are as defined above, with the proviso that, in Formula (4) above, R¹ and R² are not both F when R³ is H;
or a pharmaceutically acceptable salt thereof.

In one embodiment, R¹ is F. In a further embodiment, R² is F when R³ is H or Me. In a still further embodiment, R³ is H.

In one embodiment of the compound of Formula (3), R³ is H. In a further embodiment, R¹ is F. In a still further embodiment, R² is F. In a special embodiment, R¹ is F and R² is F.

In one embodiment of the compound of Formula (4), R³ is H. In a further embodiment, R³ is Me. In a still further embodiment, R¹ is F. In a further embodiment, R¹ is Cl. In a still further embodiment, R² is F. In a still further embodiment, R² is Cl. In a special embodiment, R¹ is F and R² is F. In a further embodiment, R¹ is Cl and R² is Cl.

In one embodiment of the compound of Formula (5), R³ is H. In a further embodiment, R¹ is F. In a still further embodiment, R² is F. In a special embodiment, R¹ is F and R² is F.

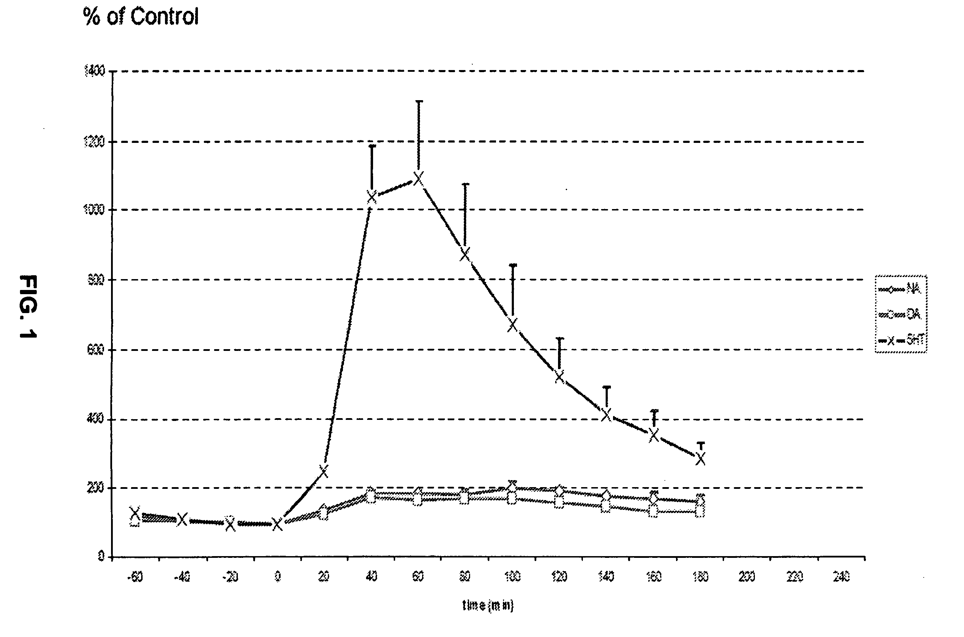

Compounds of formulae 2-6 have been found to increase the extracellular levels of norepinephrine and dopamine preferentially in the frontal cortex with no or substantially smaller effects in the striatum, as measured by the microdialysis technique. The unprecedented increase in cortical norepinephrine and dopamine of these compounds is illustrated in Figures 1-10.

Examples of compounds of the invention are:
3-(3,4-difluorophenyl)pyrrolidine;
3-(3,4-difluorophenyl)-1-methylpyrrolidine;
3-(2,4-difluorophenyl)pyrrolidine;
3-(3,5-difluorophenyl)pyrrolidine;
3-(3,4-dichlorophenyl)pyrrolidine;
3-(3-chloro-2-fluorophenyl)pyrrolidine;
3-(3-chloro-2-fluorophenyl)-1-methylpyrrolidine;
3-(2-chloro-3-fluorophenyl)pyrrolidine;
3-(2-chloro-3-fluorophenyl)-1-methylpyrrolidine;
3-(2,3-dichlorophenyl)pyrrolidine;
3-(2,3-dichlorophenyl)-1-methylpyrrolidine;
3-(2,3-difluorophenyl)pyrrolidine;
3-(2,3-difluorophenyl)-1-methylpyrrolidine;
3-(4-chloro-2-fluorophenyl)pyrrolidine;
3-(4-chloro-2-fluorophenyl)-1-methylpyrrolidine;
3-(2-chloro-4-fluorophenyl)pyrrolidine;
3-(2-chloro-4-fluorophenyl)-1-methylpyrrolidine;
3-(2,4-dichlorophenyl)pyrrolidine;
3-(2,4-dichlorophenyl)-1-methylpyrrolidine;
3-(2,4-d ifluorophenyl)-1-methylpyrrolidine;
3-(4-chloro-3-fluorophenyl)pyrrolidine;
3-(4-chloro-3-fluorophenyl)-1-methylpyrrolidine;
3-(3-chloro-4-fluorophenyl)pyrrolidine;
3-(3-chloro-4-fluorophenyl)-1-methylpyrrolidine;
3-(3,4-dichlorophenyl)-1-methylpyrrolidine;
3-(3-chloro-5-fluorophenyl)pyrrolidine;
3-(3-chloro-5-fluorophenyl)-1-methylpyrrolidine;
3-(3,5-dichlorophenyl)pyrrolidine;
3-(3,5-dichlorophenyl)-1-methylpyrrolidine; and
3-(3,5-difluorophenyl)-1-methylpyrrolidine.

Any combination of two or more of the embodiments as described above is considered within the scope of the present invention.

### Pharmaceutically Acceptable Salts

The chemical compound of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate, the phthalate, the salicylate, the sorbate, the stearate, the succinate, the tartrate, the toluene-p-sulphonate, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Examples of pharmaceutically acceptable cationic salts of a chemical compound of the invention include, without limitation, the sodium, the potassium, the calcium, the magnesium, the zinc, the aluminium, the lithium, the choline, the lysinium, and the ammonium salt, and the like, of a chemical compound of the invention containing an anionic group. Such cationic salts may be formed by procedures well known and described in the art.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts. Examples of pre- or prodrug forms of the chemical compound of the invention include examples of suitable prodrugs of the substances according to the invention include compounds modified at one or more reactive or derivatizable groups of the parent compound. Of particular interest are compounds modified at a carboxyl group, a hydroxyl group, or an amino group. Examples of suitable derivatives are esters or amides.

Specific examples of prodrugs of the compounds of the present invention are the N-oxides mentions below and the following N-hydroxy-derivatives:
3-(3,5-difluorophenyl)pyn-olidin-1-ol;
3-(3-chloro-2-fluorophenyl)pyrrolidin-1-ol;
3-(2-chloro-3-fluorophenyl)pyrrolidin-1-ol;
3-(2,3-dichlorophenyl)pyrrolidin-1-ol;
3-(2,3-difluorophenyl)pyrrolidin-1-ol;
3-(2-chloro-4-fluorophenyl)pyrrolidin-1-ol;
3-(2,4-dichlorophenyl)pyrrolidin-1-ol;
3-(2,4-difluorophenyl)pyrrolidin-1-ol;
3-(4-chloro-2-fluorophenyl)pyrrolidin-1-ol;
3-(4-chloro-3-fluorophenyl)pyrrolidin-1-ol;
3-(3-chloro-4-fluorophenyl)pyrrolidin-1-ol;
3-(3,4-dichlorophenyl)pyrrolidin-1-ol;
3-(3,4-difluorophenyl)pyrrolidin-1-ol;
3-(3-chloro-5-fluorophenyl)pyrrolidin-1-ol; and
3-(3,5-dichlorophenyl)pyrrolidin-1-ol.

The chemical compound of the invention may be provided in dissoluble or indissoluble forms together with a pharmaceutically acceptable solvent such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

### Steric Isomers

It will be appreciated by those skilled in the art that the compounds of the present invention may exist in different stereoisomeric forms.

The invention includes all such isomers and any mixtures thereof including racemic mixtures.

Racemic forms can be resolved into the optical antipodes by known methods and techniques. One way of separating the enantiomeric compounds (including enantiomeric intermediates) is - in the case the compound being a chiral acid - by use of an optically active amine, and liberating the diastereomeric, resolved salt by treatment with an acid. Another method for resolving racemates into the optical antipodes is based upon chromatography on an optical active matrix. Racemic compounds of the present invention can thus be resolved into their optical antipodes, e.g., by fractional crystallisation of D- or L- (tartrates, mandelates, or camphorsulphonate) salts for example.

The chemical compounds of the present invention may also be resolved by the formation of diastereomeric amides by reaction of the chemical compounds of the present invention with an optically active carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### N-oxides

In the context of this invention an N-oxide designates an oxide derivative of a tertiary amine, including a nitrogen atom of an aromatic N-heterocyclic compound, a nonaromatic N-heterocyclic compounds, a trialkylamine and a trialkenylamine. For example, the N-oxide of a compound containing a pyridyl may be the 1-oxy-pyridin-2, -3 or -4-yl derivative.

N-oxides of the compounds of the invention may be prepared by oxidation of the corresponding nitrogen base using a conventional oxidizing agent such as hydrogen peroxide in the presence of an acid such as acetic acid at an elevated temperature, or by reaction with a peracid such as peracetic acid in a suitable solvent, e.g. dichloromethane, ethyl acetate or methyl acetate, or in chloroform or dichloromethane with 3-chloroperoxybenzoic acid.

The following N-oxides act as prodrugs to the compounds of the invention:
Formula (7): 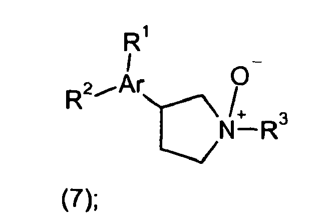
wherein Ar is phenyl;
R¹ is selected from the group consisting of F and Cl;
R² is selected from the group consisting of F and Cl;
R³ is selected from the group consisting of H and Me;
and the pharmaceutically acceptable salts thereof.

Of particular interest are prodrugs having Formula (8): 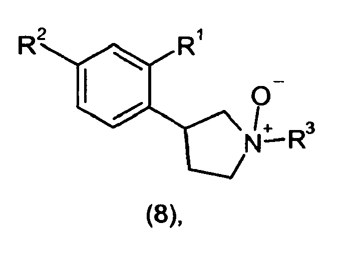 or Formula (9): 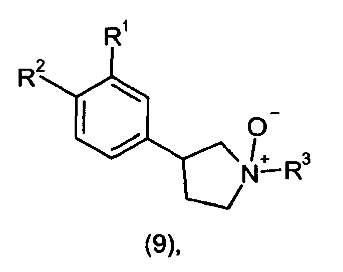 or Formula (10): 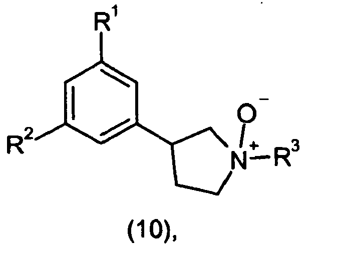 or Formula (11): 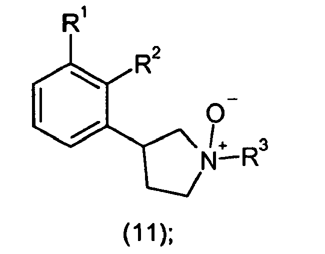 wherein:
R¹ is selected from the group consisting of F and Cl;
R² is selected from the group consisting of F and Cl;
R³ is selected from the group consisting of H and Me;
and the pharmaceutically acceptable salts thereof.

Examples of N-oxides according to the invention are:
3-(3-chloro-2-fluorophenyl)-1-methylpyrrolidine 1-oxide;
3-(2,3-dichlorophenyl)-1-methylpyrrolidine 1-oxide;
3-(2,3-difluorophenyl)-1-methylpyrrolidine 1-oxide;
3-(2-chloro-3-fluoropheny))-1-methylpyrrolidine 1-oxide;
3-(4-chloro-2-fluorophenyl)-1-methylpyrrolidine 1-oxide;
3-(2,4-dichlorophenyl)-1-methylpyrrolidine 1-oxide;
3-(2,4-difluorophenyl)-1-methylpyrrolidine 1-oxide;
3-(2-chloro-4-fluorophenyl)-1-methylpyrrolidine 1-oxide;
3-(4-chloro-3-fluorophenyl)-1-methylpyrrolidine 1-oxide;
3-(3,4-dichlorophenyl)-1-methylpyrrolidine 1-oxide;
3-(3-chloro-4-fluorophenyl)-1-methylpyrrolidine 1-oxide;
3-(3,4-difluorophenyl)-1-methylpyrrolidine 1-oxide;
3-(3-chloro-5-fluorophenyl)-1-methylpyrrolidine 1-oxide;
3-(3,5-dichlorophenyl)-1-methylpyrrolidine 1-oxide; and
3-(3,5-difluorophenyl)-l-methylpyrrolidine 1-oxide.

### Labelled Compounds

The compounds of the invention may be used in their labelled or unlabelled form. In the context of this invention the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from ²H (deuterium), ³H (tritium), ¹¹C, ¹³C, ¹⁴C, ¹³¹I, ¹²⁵I, ¹²³I, and ¹⁸F.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

### Biological Activity

The compounds according to the present invention possess norepinephrine, dopamine and to some extent serotonin-modulating properties and both they and their pharmaceutical compositions are useful in treating numerous central nervous system disorders including psychiatric disorders. Particularly, the compounds and their pharmaceutical compositions can be used in the treatment of CNS disorders where the cortical monoaminergic systems are dysfunctional due to direct or indirect causes.

The compounds and compositions according to the invention can be used to treat cognitive disorders including neurodegenerative (e.g. dementia and age-related cognitive impairment) and developmental disorders, such as Autism spectrum disorders, ADHD, Cerebral Palsy, Gilles de la Tourette's syndrome, as well as cognitive disorders occurring as part of the core symptoms of schizophrenia.

The compounds and compositions according to the invention can be used to treat affective disorders including depression and bipolar disorder. They can also be used to treat schizophrenia and schizophreniform disorders.

The compounds and compositions according to the invention can be used to treat anxiety disorders including generalized anxiety disorder (GAD), specific phobias and panic disorder (PD). They are also useful for treatment of sleep disorders.

The compounds according to the present invention have been shown to increase the extra-cellular levels of dopamine and norepinephrine in the cerebral cortex and in some cases also serotonin.

However, compounds of the present invention do not have the effects on the metabolism of dopamine in the striatum that are characteristic for the pharmacological actions of the compounds described in WO 01/46146, WO 01/46145, WO 92/18475 J. Med. Chem. 1994, 37, 2735 or Bioorg. Med. Chem. Lett. 1997. Compounds of the present invention have a surprising and distinct pharmacology (see Table 1).

**Table 1: The increase in DOPAC levels (3,4-dihydroxyphenylacetic acid) in the rat striatum after systemic adminstration of test compound (100 µmol/kg s.c.). Expressed as the %-increase from control value. For method see the enclosed description.**

| **Comparative Examples¹** | **DOPAC %-increase** |
|---|---|
| Example 10 of ref. 1 | + 262² |
| Example 16 of ref. 1 | + 150² |
| Example 26 of ref. 1 | + 67² |
| Example 27 of ref. 1 | + 74² |
| Example 10d of ref. 2 | + 63³ |
| Example 12d of ref. 2 | + 197³ |
| Example 9 of WO01/46146 | + 94 |
| Example 43 of WO01/46146 | + 94 |
| Example 44 of WO01/46146 | + 239 |
| Claimed in WO01/46146 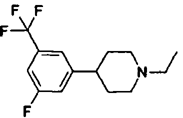 | + 232 |
| Claimed in WO01/46146 | + 75 |
| 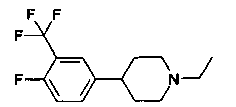 | |
| Example 6 of WO01/46145 | + 114 |

| **Examples** | **DOPAC %increase** |
|---|---|
| Example 1 | - 45 |
| Example 2 | - 22 |
| Example 3 | - 19 |
| Example 4 | - 20 |
| Example 5 | - 37 |

| | |
|---|---|
| *¹Comparative Examples from prior art; Ref 1:* J. Med. Chem. 1994, 37, 2735*; Ref 2:* Bioorg. Med. Chem. Lett. 1997, 7, 241-246*. ²Data taken from Table 2 in Ref 1. ³Data taken from Table 2 in Ref 2. The data from this reference is DOPA accumulation and not DOPAC. DOPAC and DOPA are both a measure of the indirect change in concentration of dopamine in the brain of the experimental animals. An increase in DOPAC and DOPA levels show an increased synthesis and turnover of dopamine in the system. DOPA accumulation measures the increase in the concentration of 3,4-dihydroxyphenylalanine in the striatal regions of the brain. DOPAC measures the increase in the concentration of 3,4-dihydroxy phenylacetic acid in the striatal regions of the brain. There is a strong relation between DOPA and DOPAC.* | |

It can be seen that - upon administration - the known compounds tested produce a significant increase in striatum DOPAC levels. In contrast, compounds of the present invention have surprisingly shown to provide a decrease in striatum DOPAC levels. On the other hand, the essential characteristic of compounds of the present invention is to produce increased cortical levels of catecholamines, measured as the extracellular levels of dopamine and norepinephrine assessed by the microdialysis technique, while displaying no or at most weak effects on subcortical catecholamines (FIGURES 1-10).

### Description of animal models used in the invention

The measurement of the tissue content of DOPAC is well established in the field of research since the 1960's. In short, male Sprague-Dawely rats are administered the test compound 60 minutes prior to decapitation. The brain is rapidly taken out and dissected. The striatum is rapidly frozen and subsequenlty quantitatively analysed with respect to its content of DOPAC by means of HPLC and electrochemical detection. The number of animals used for each test compound/vehicle is 4/group.

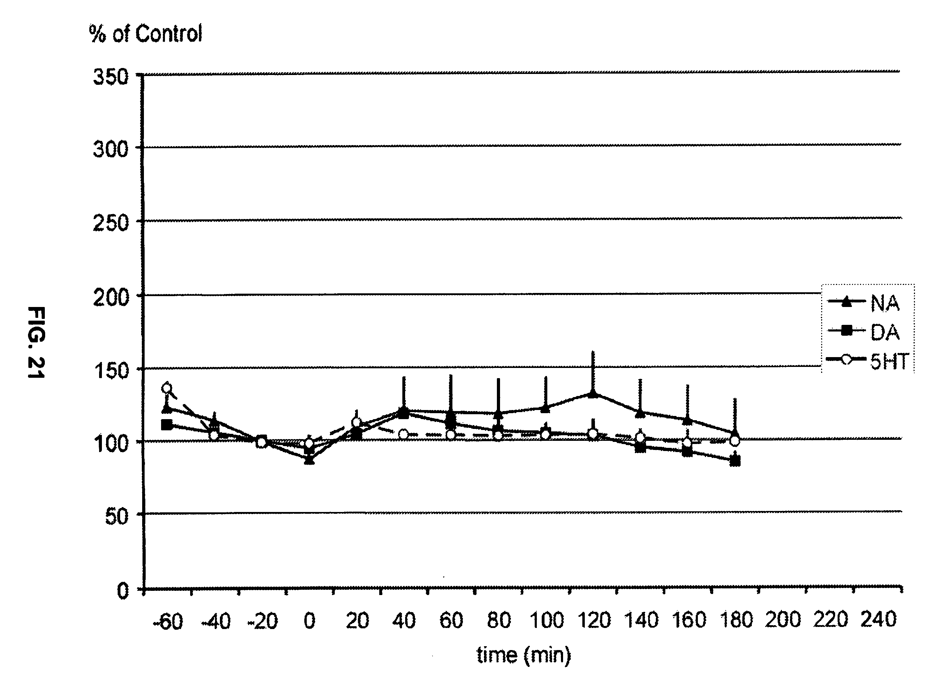

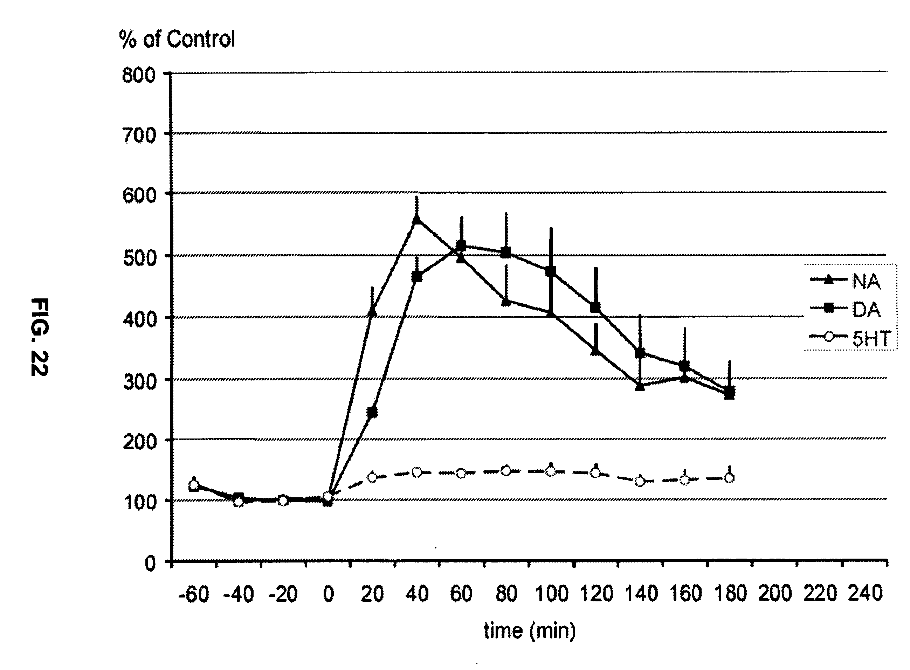

The microdialysis technique (Ungerstedt, Herrera-Marschitz et al. 1982) is a well established technique for measuring extracellular levels of neurotransmitters (Ungerstedt 1991). The microdialysis technique was used to measure the effect of drugs upon the monoamine transmitters. The appended graphs (Figures 21 and 22) show the effects of one established antidepressant (mirtazapine) upon monoamines in the striatum and frontal cortex, as well as for compounds claimed in the present invention (Figures 1-10; Examples 1-5). The number of animals (n) used for each compound tested is noted in the figure legend.

### Effects on dopamine and norepinephrine in cortical regions

### Cognition

The cortical circuitry underlying cognitive functions including memory, attention and working memory comprises a network of glutamatergic and GABAergic neurons, innervated by ascending dopaminergic and norepinephrinergic projections (Harrison and Weinberger 2005, Arnsten and Li 2005). Dopamine, acting through DA D1 receptors, enhances cognitive functions, while hypofunction of the cortical DA transmission produces specific cognitive deficits (reviewed in Goldman-Rakic, 2004). Likewise, norepinephrine has been found to enhance cognitive functions, presumably depending on stimulation of post-synaptic alpha-2 receptors in the prefrontal cortex (Arnsten, 2004). Clinical examples of the effects of cortical DA and NE deficiency are the cognitive disorders seen in schizophrenia and ADHD. In schizophrenia, cortical DA deficiency is regarded as a key feature underlying cognitive dysfunctions (Perlman et al., 2004, Goldman-Rakic, 2004). One mechanism by which such cortical DA hypofunction is believed to arise is a well described point mutation in the COMT encoding gene, leading to exagerrated activity of COMT, and therefore, an increased rate of elimination of DA, and ensuing, decreased levels of DA particularly in the cortex (Harrison and Weinberger 2005, Perlman et al., 2004). This mutation of COMT is genetically linked to schizophrenia as well as correlated to cognitive performance in healthy individuals. Apart from COMT anomalies, a variety of other pathogenetic pathways are proposed to lead to a functionally similar state of cortical dysfunction in schizophrenia, manifested by the characteristic abnormalities of cognitive functions seen in schizophrenic patients (Harrison and Weinberger, 2005). For instance, a number of susceptibility genes are thought to preferentially affect NMDArecptor mediated glutamate transmission. Due to the beneficial effects on cognitive functions by augmented DA D1 receptor stimulation, strengthening of cortical DA transmission can normalise cortical activity and enhance cognitive functions in schizophrenia as well as in other conditions (Goldman-Rakic, 2004). Furthermore, since the abnormalities in the cortical microcircuitry are regarded as the core feature underlying the clinical syndrome, restoration of this microcircuitry by facilitating DA transmission should not only improve cognitive functions in schizophrenia, but also reduce psychotic symptoms. Thus, normalisation of cortical DA transmission would as a secondary effect lead to normalisation of subcortical DA transmission, and thus, alleviation of the symptoms related to subcortical hyperdopaminergia (Goldman-Rakic, 2004, Perlman et al., 2004). Furthermore, a common feature of atypical antipsychotics, hypothesised to underlye their superior efficacy and fewer side effects compared to other antipsychotic compounds, is their ability to increase cortical dopamine (Moghaddam and Bunney, 1990, Deutch et al., 1991). It is important to note that the principle described in this invention to achieve cognitive enhancement and antipsychotic effects is dependent on regionally selective cortical increase in DA and NE, while increases in subcortical, eg striatal, DA are not sought for. In conclusion, compounds according to this invention that increase cortical DA, but not subcortical DA transmission, will improve cognitive functions and reduce psychotic symptoms in schizophrenia.

The other clinical example showing the role of DA and NE in cognitive functions is the clinical features of ADHD, including the mode of action of compounds used to relieve the symptoms in this disorder. The key features of ADHD are deficiencies in attention, lack of ability to focus on a task for a prolonged time, impulsivity, and hyperactivity (Biederman 2005, Arnsten and Li 2005). In neuropsychological tests, ADHD patients perform poorly on tests specifically assessing prefrontal cortical functions (Arnsten and Li, 2005). The structure of the cortical circuitry underlying these functions suggests that insufficient DA and NE transmission would lead to the specific neuropsychological deficits seen in ADHD. Studies on the etiology of ADHD all point toward disregulation of DA and NE, particularly in cortical regions. The pharmacological treatments available are mainly psycho-stimulants, including dex-amphetamine, and methylphenidate, which increase DA and NE in most brain areas. A recent advancement in the treatment of ADHD is the compound atomoxetine (US 5,658,590), which produces regionally selective increases in cortical DA and NE, relieving core symptoms while avoiding side effects related to increase subcortical in DA transmission, thus supporting that cortical, rather than subcortical effects on catecholamines are essential to the clinical efficacy of ADHD medications (Pliszka, 2005).

Taken together, there is solid evidence that enhanced cortical DA and NE transmission would improve the symptoms of ADHD, including cognitive improvement. Furthermore, the role of cortical DA and NE in cognitive functions implies that enhancement of cortical DA transmission also improves cognitive functioning in cognitive disorders arising from causes other than schizophrenia or ADHD, as well as in healthy individuals. This is supported by the correlation between COMT activity and cognitive performance in healthy individuals (Perlman et al., 2004) and by numerous studies in rodents, primates and humans concerning the influence of cortical DA and NE on cognitive functions in healthy states as well as in different disorders (Arnsten, 2004, Goldman-Rakic, 2004). Consequently, the compounds according to the present invention will be useful to treat the symptoms of ADHD, as well as cognitive disorders in general, due to their ability to produce regionally selective increases in cortical DA and NE.

### Anxiolytic and antidepressant actions

A common trait for all clinically effective classes of antidepressants is an elevation of the levels of dopamine and norepinephrine in the cortex (Tanda, Carboni et al. 1994; Millan, Lejeune et al. 2000). As an example, the clinically effective antidepressant mirtazapine (remeron) has been shown to increase predominantly extracellullar norepinephrine and dopamine in the cortex (See Figures 21 and 22, and Devoto, Flore et al. 2004). As compounds claimed in the present invention elevate the levels of dopamine and norepinephrine in the cortex this supports our claim that they function as antidepressants (see Figures 1-10, Examples 1-5 in the present invention). Furthermore, norepinephrine is strongly involved in the neuronal pathways, comprising the locus ceruleus, the amygdala, and the cerebral cortex, controlling fear and anxiety and so, modulation of cortical norepinephrine transmission modulates states of anxiety (Sullivan et al. 1999, Biol Psychiatry;46:1205-121). Accordingly, compounds that alter cortical norepinephrinergic transmission are reported to be effective in the treatment of anxiety disorders. More specifically, NE modulating compounds like mirtazapine (Remeron), which produces marked increases in cortical NE levels by a mechanism other than NE reuptake inhibition (Fig 22), and venlafaxine, which increases cortical NE by inhibition of norepinephrine re-uptake, both have anxiolytical properties in clinical studies (Neuropsychopharmacology, 5th generation of Progress, Lippincott, Williams and Wilkins 2002, pp 967 - 980). Based on this evidence for the beneficial effects of enhanced cortical norepinephrine transmission on anxiety disorders, along with the neurobiological back-ground demonstrating the crucial role of norepinephrine in the control of anxiety, it is concluded that the compounds of the present invention, which produces marked increases in cortical NE will be effective in the treatment of anxiety disorders.

### BRIEF DESCRIPTION OF THE DRAWINGS

The present invention is further illustrated by reference to the accompanying drawings.

### Examples according to the invention (Figures 1-10)

### Figure 1. Example 1, 50 µmol/kg s.c. striatum amines

Example 1 is injected (s.c.) at time-point 0. The values depicted in Figure 1 represent percent of control in relation to baseline values. The microdialysis was performed in awake and freely moving rats. Dopamine = DA; Norepinephrine = NA; Serotonin = 5-HT; Error-bars=SEM

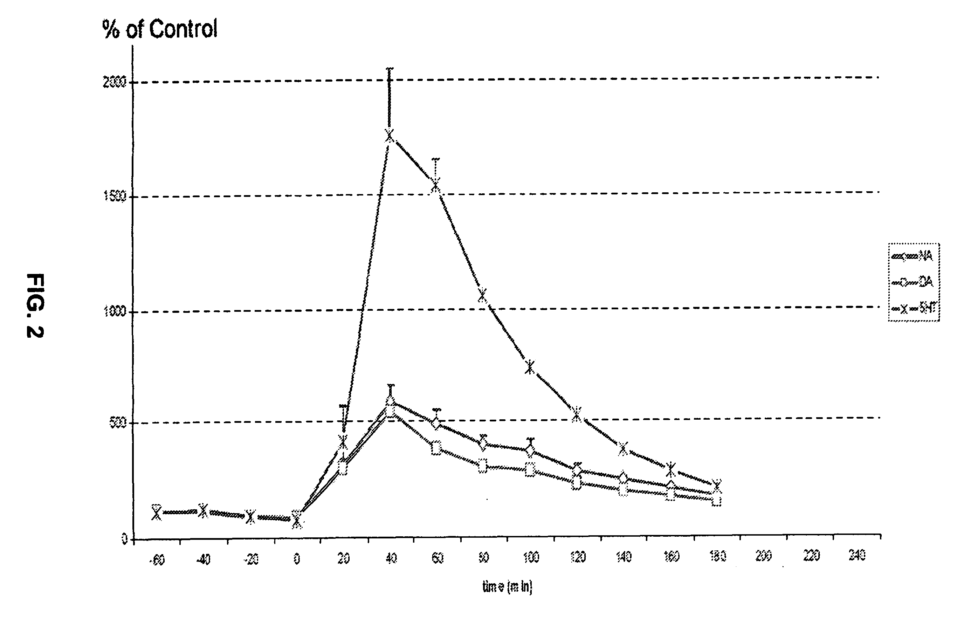

### Figure 2, Example 1, 50 µmol/kg s.c. p.f. cortex amines

Example 1 is injected (s.c.) at time-point 0. The values depicted in Figure 2 represent percent of control in relation to baseline values. The microdialysis was performed in awake and freely moving rats. Dopamine = DA; Norepinephrine = NA; Serotonin = 5-HT; Error-bars=SEM

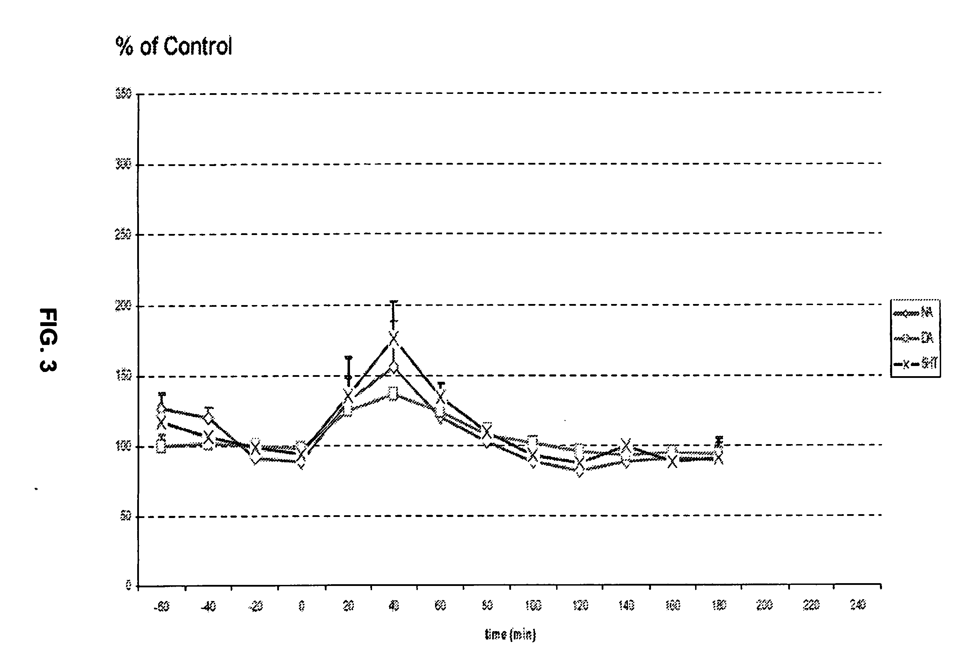

### Figure 3. Example 2, 50 µmol/kg s.c. striatum amines

Example 2 is injected (s.c.) at time-point 0. The values depicted in Figure 3 represent percent of control in relation to baseline values. The microdialysis was performed in awake and freely moving rats. Dopamine = DA; Norepinephrine = NA; Serotonin = 5-HT; Error-bars=SEM

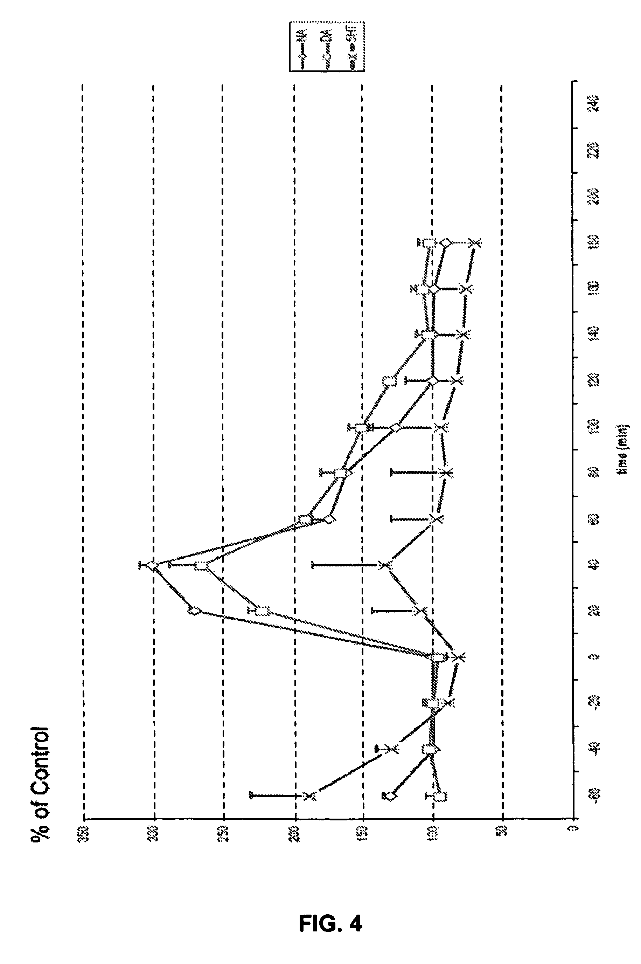

### Figure 4. Example 2, 50 µmol/kg s.c. p.f. cortex amines

Example 2 is injected (s.c.) at time-point 0. The values depicted in Figure 4 represent percent of control in relation to baseline values. The microdialysis was performed in awake and freely moving rats. Dopamine = DA; Norepinephrine = NA; Serotonin = 5-HT; Error-bars=SEM

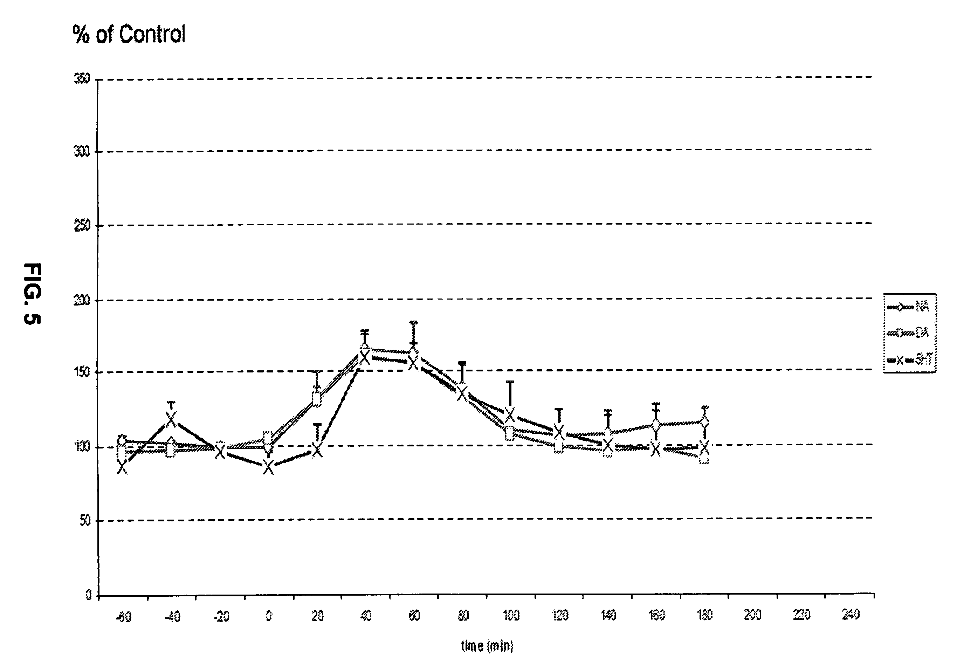

### Figure 5. Example 3, 50 µmol/kg s.c. striatum amines

Example 3 is injected (s.c.) at time-point 0. The values depicted in Figure 5 represent percent of control in relation to baseline values. The microdialysis was performed in awake and freely moving rats. Dopamine = DA; Norepinephrine = NA; Serotonin = 5-HT; Error-bars=SEM

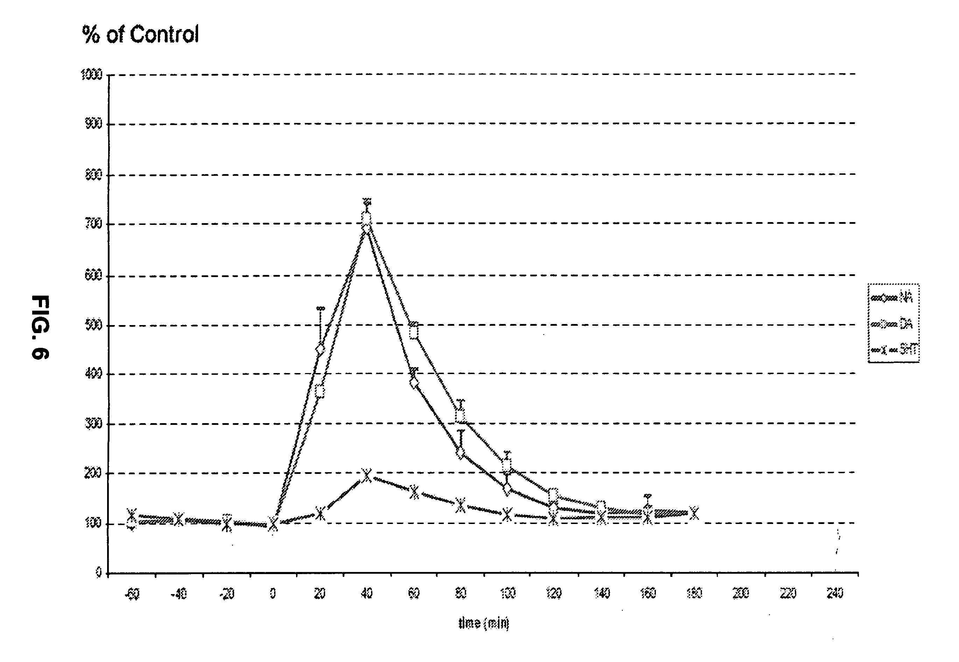

### Figure 6. Example 3, 50 µmol/kg s.c. p.f. cortex amines

Example 3 is injected (s.c.) at time-point 0. The values depicted in Figure 6 represent percent of control in relation to baseline values. The microdialysis was performed in awake and freely moving rats. Dopamine = DA; Norepinephrine = NA; Serotonin = 5-HT; Error-bars=SEM

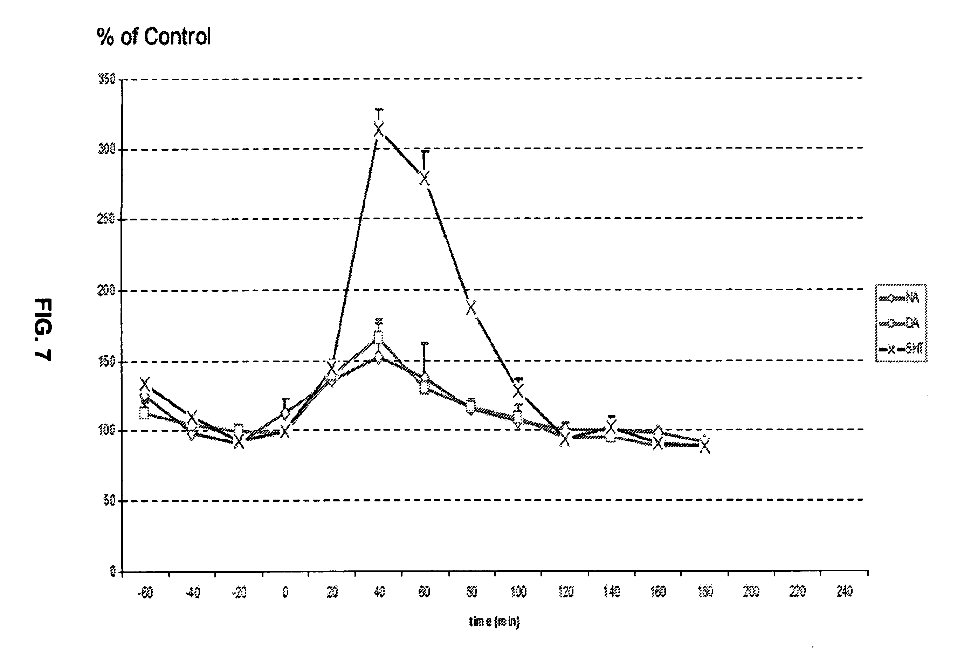

### Figure 7. Example 4, 50 µmol/kg s.c. striatum amines

Example 4 is injected (s.c.) at time-point 0. The values depicted in Figure 7 represent percent of control in relation to baseline values. The microdialysis was performed in awake and freely moving rats. Dopamine = DA; Norepinephrine = NA; Serotonin = 5-HT; Error-bars=SEM

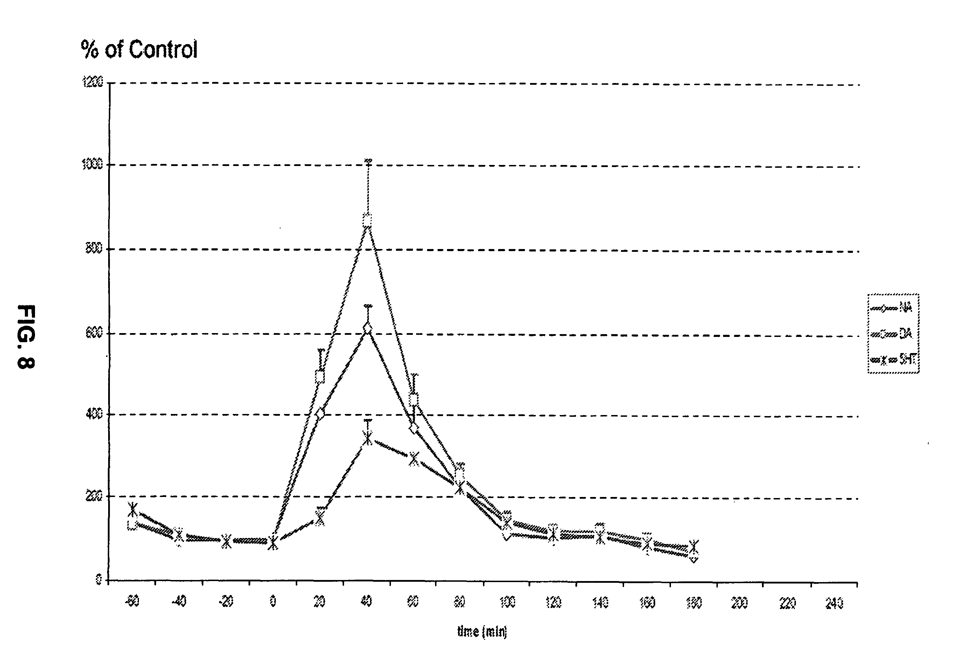

### Figure 8. Example 4, 50 µmol/kg s.c. pf. cortex amines

Example 4 is injected (s.c.) at time-point 0. The values depicted in Figure 8 represent percent of control in relation to baseline values. The microdialysis was performed in awake and freely moving rats. Dopamine = DA; Norepinephrine = NA; Serotonin = 5-HT; Error-bars=SEM

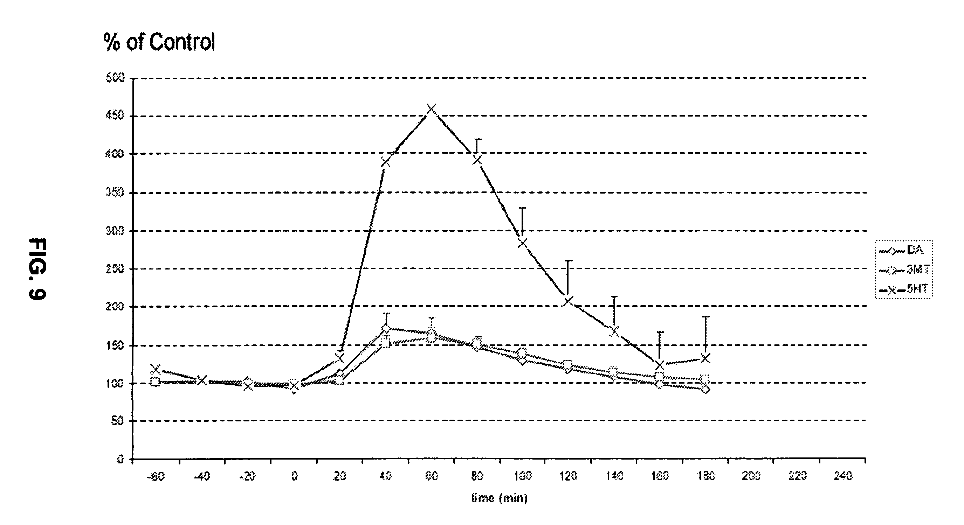

### Figure 9. Example 5, 50 µmol/kg s.c. striatum amines

Example 5 is injected (s.c.) at time-point 0. The values depicted in Figure 9 represent percent of control in relation to baseline values. The microdialysis was performed in awake and freely moving rats. Dopamine = DA; Norepinephrine = NA; Serotonin = 5-HT; Error-bars=SEM

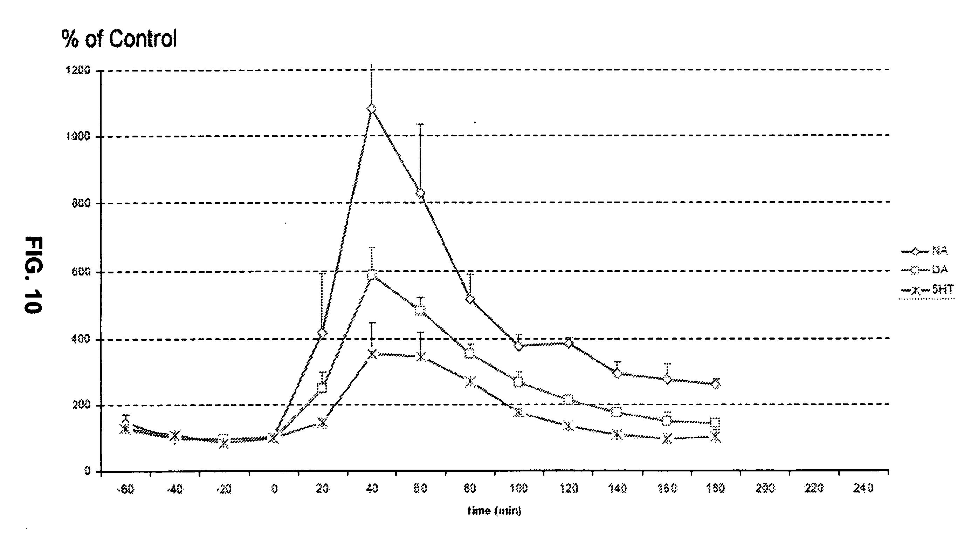

### Figure 10. Example 5, 50 µmol/kg s.c. p.f. cortex amines

Example 3 is injected (s.c.) at time-point 0. The values depicted in Figure 10 represent percent of control in relation to baseline values. The microdialysis was performed in awake and freely moving rats. Dopamine = DA; Norepinephrine = NA; Serotonin = 5-HT; Error-bars=SEM

### Comparative Examples (Figures 11-22)

### Figure 11. (S)-(-)-3-[3-methylsulfonyl)phenyl]-1-propylpiperidine (Example 16 in J. Med. Chem. 1994, 37, 2735) 50µmol/kg s.c. p.f. striatum

(S)-(-)-3-[3-methylsulfonyl)phenyl]-1-propylpiperidine is injected (s.c) at time-point 0. The values depicted in Figure 11 represent percent of control in relation to baseline values. The microdialysis was performed in awake and freely moving rats. Dopamine = DA; Norepinephrine = NA; Serotonin = 5-HT Error-bars=SEM

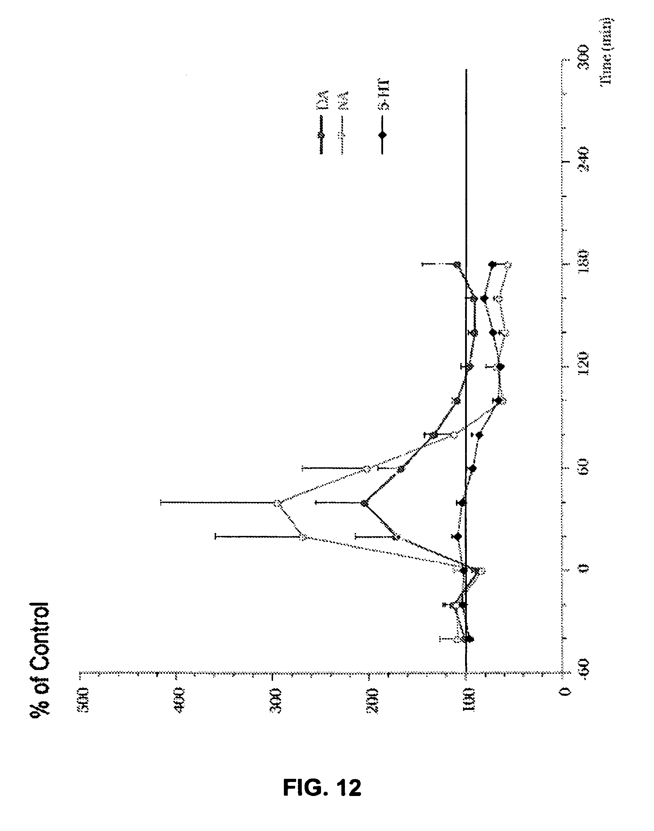

### Figure 12. (S)-(-)-3-[3-methylsulfonyl)phenyl]-1-propylpiperidine (Example 16 in J. Med. Chem. 1994, 37, 2735) 50 µmol/kg s.c. p.f. cortex

(S)-(-)-3-[3-methylsulfonyl)phenyl]-1-propylpiperidine is injected (s.c) at time-point 0. The values depicted in Figure 12 represent percent of control in relation to baseline values. The microdialysis was performed in awake and freely moving rats. Dopamine = DA; Norepinephrine = NA; Serotonin = 5-HT Error-bars=SEM

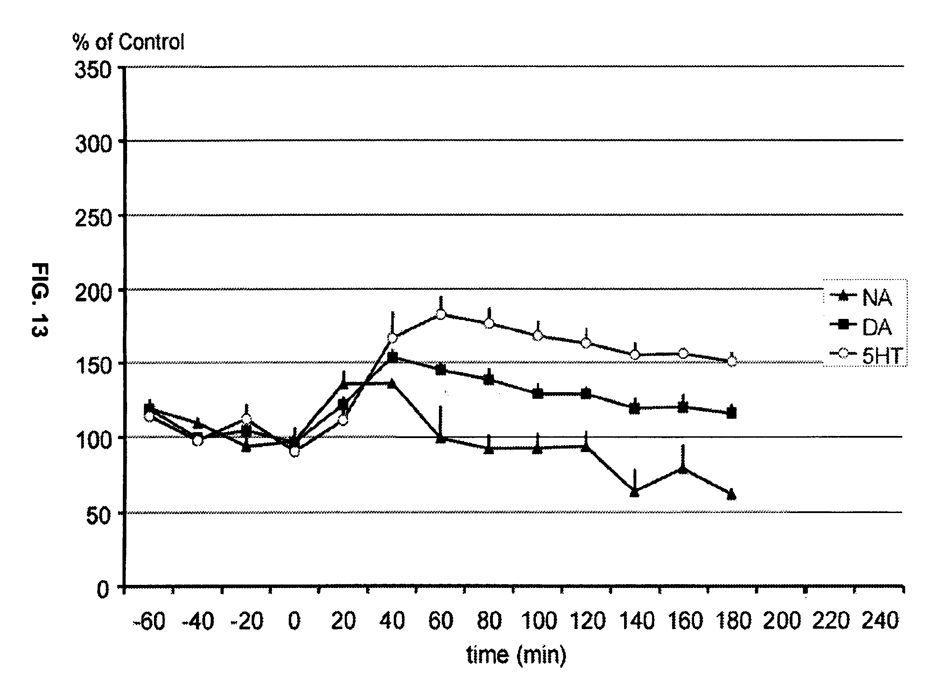

### Figure 13. 4-(4-chloro-3-trifluoromethyl-phenyl)-1-propyl-piperidine (Example 9 in WO 01/46146) 50 µmol/kg s.c. striatum amines

4-(4-chloro-3-trifluoromethyl-phenyl)-1-propyl-piperidine is injected (s.c) at time-point 0. The values depicted in Figure 13 represent percent of control in relation to baseline values. The microdialysis was performed in awake and freely moving rats. Dopamine = DA; Norepinephrine = NA; Serotonin = 5-HT; Error-bars=SEM

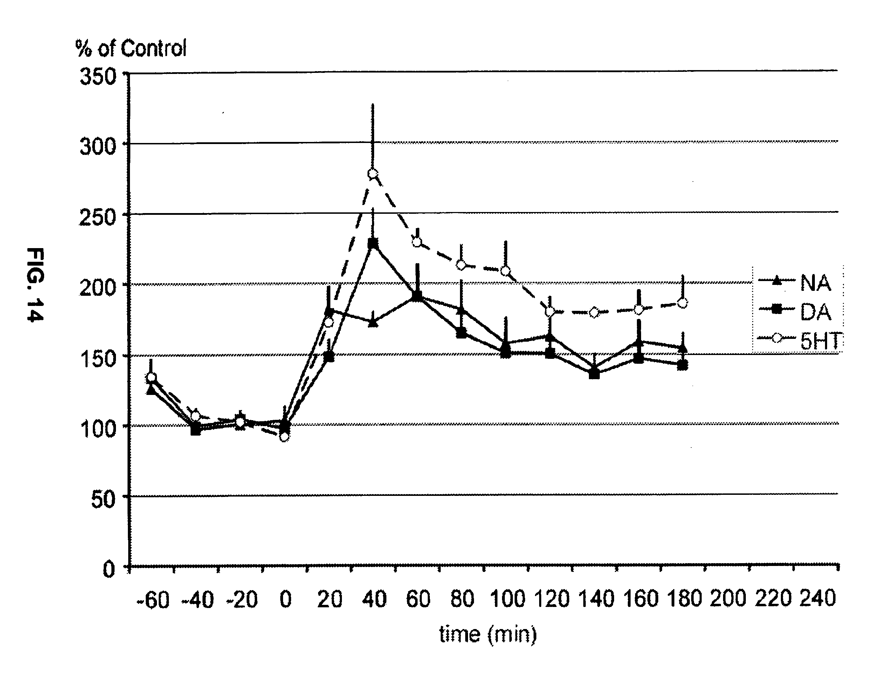

### Figure 14. 4-(4-chloro-3-trifluoromethyl-phenyl)-1-propyl-piperidine (Example 9 in WO 01/46146) 50 µmol/kg s.c. p.f. cortex

4-(4-chloro-3-trifluoromethyl-phenyl)-1-propyl-piperidine is injected (s.c) at time-point 0. The values depicted in Figure 14 represent percent of control in relation to baseline values. The microdialysis was performed in awake and freely moving rats. Dopamine = DA; Norepinephrine = NA; Serotonin = 5-HT Error-bars=SEM

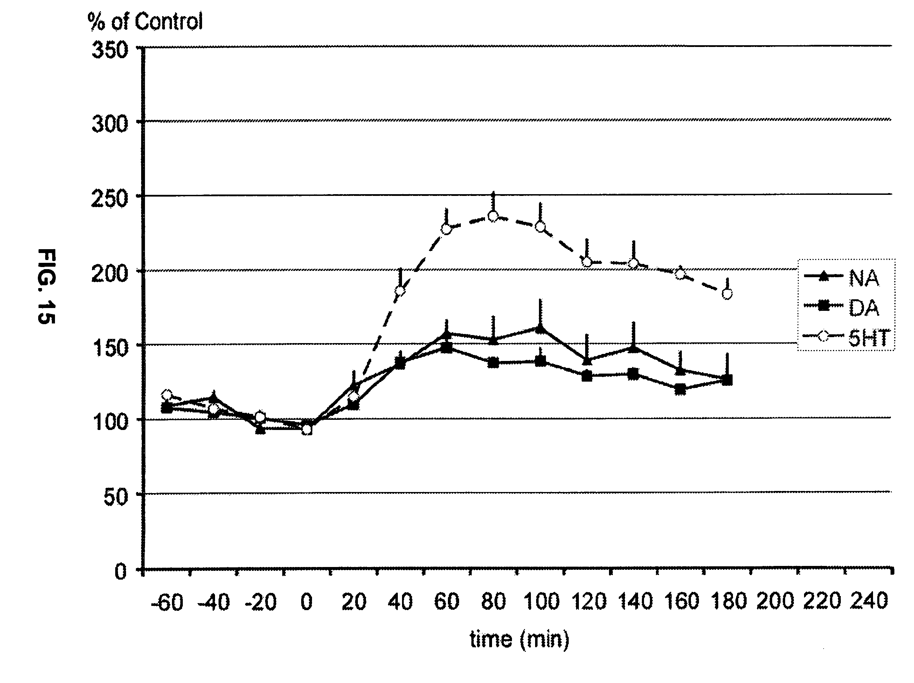

### Figure 15. 4-(4-fluoro-3-trifluoromethyl-phenyl)-1-ethyl-piperidine (claimed in WO 01/46146) 50 µmol/kg s.c. striatum amines

4-(4-fluoro-3-trifluoromethyl-phenyl)-1-ethyl-piperidine is injected (s.c) at time-point 0. The values depicted in Figure 15 represent percent of control in relation to baseline values. The microdialysis was performed in awake and freely moving rats. Dopamine = DA; Norepinephrine = NA; Serotonin = 5-HT; Error-bars=SEM

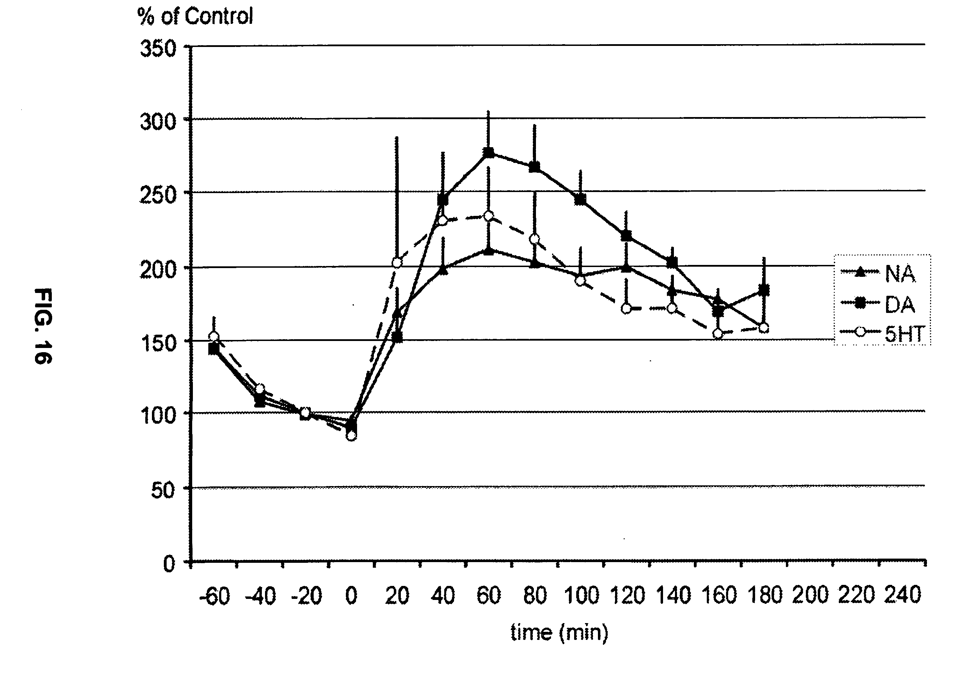

### Figure 16. 4-(4-fluoro-3-trifluoromethyl-phenyl)-1-ethyl-piperidine (claimed in WO 01/46146) 50 µmol/kg s.c.. p.f. cortex

4-(4-fluoro-3-trifluoromethyl-phenyl)-1-ethyl-piperidine is injected (s.c) at time-point 0. The values depicted in Figure 16 represent percent of control in relation to baseline values. The microdialysis was performed in awake and freely moving rats. Dopamine = DA; Norepinephrine = NA; Serotonin = 5-HT; Error-bars=SEM

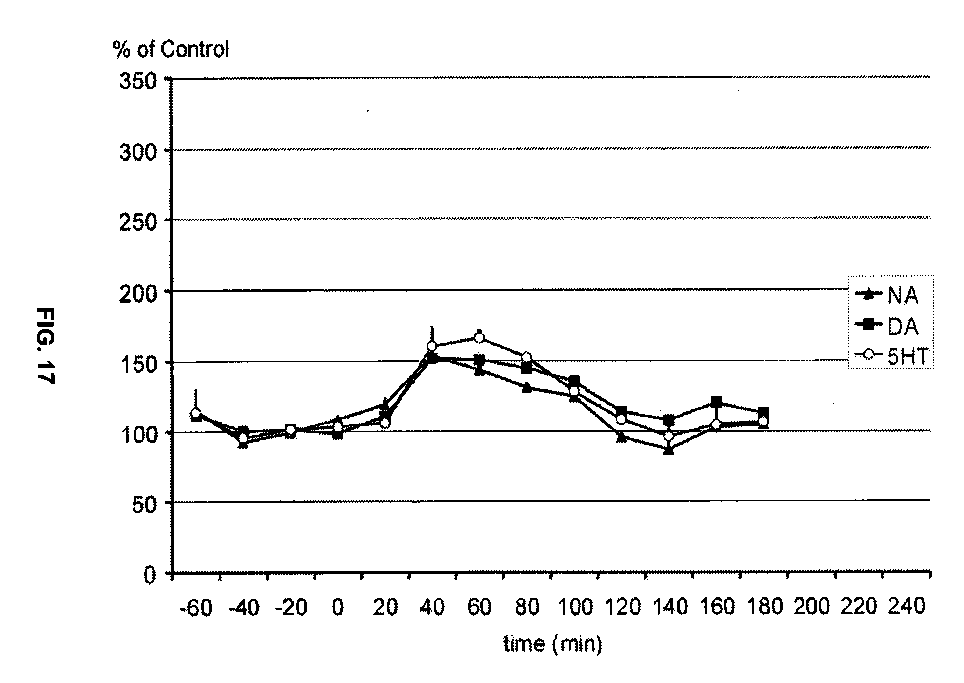

### Figure 17. 4-(3-fluoro-5-trifluoromethyl-phenyl)-1-propyl-piperidine (Example 44 in WO 01/46146) 50 µmol/kg s.c. striatum amines

4-(3-fluoro-5-trifluoromethyl-phenyl)-1-propyl-piperidine is injected (s.c) at time-point 0. The values depicted in Figure 17 represent percent of control in relation to baseline values. The microdialysis was performed in awake and freely moving rats. Dopamine = DA; Norepinephrine = NA; Serotonin = 5-HT; Error-bars=SEM

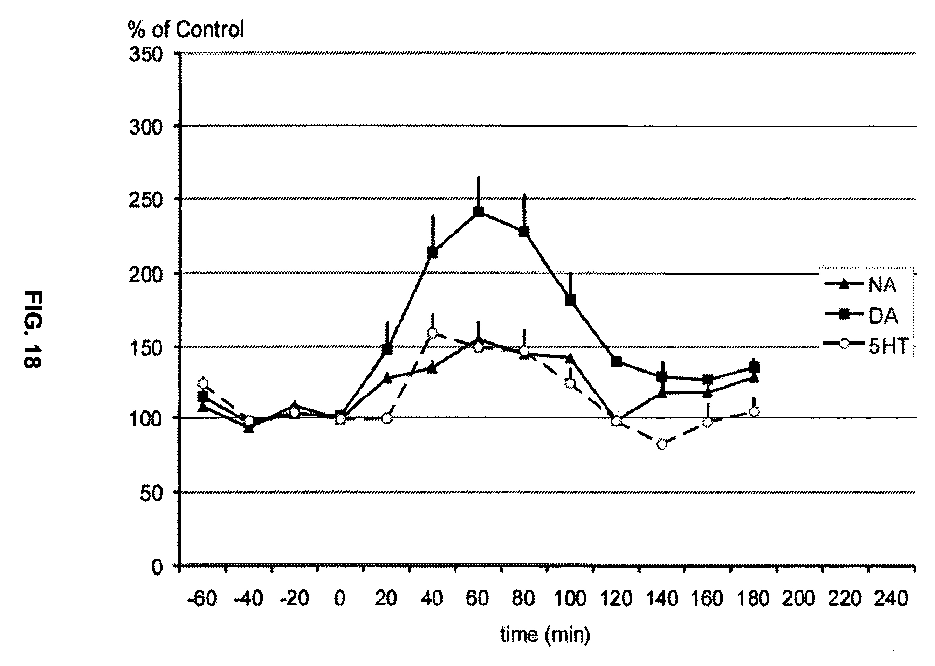

### Figure 18. 4-(3-fluoro-5-trifluoromethyl-phenyl)-1-propyl-piperidine (Example 44 in WO 01/46146) 50 µmol/kg s.c. p.f. cortex

4-(3-fluoro-5-trifluoromethyl-phenyl)-1-propyl-piperidine is injected (s.c) at time-point 0. The values depicted in Figure 18 represent percent of control in relation to baseline values. The microdialysis was performed in awake and freely moving rats. Dopamine = DA; Norepinephrine = NA; Serotonin = 5-HT; Error-bars=SEM

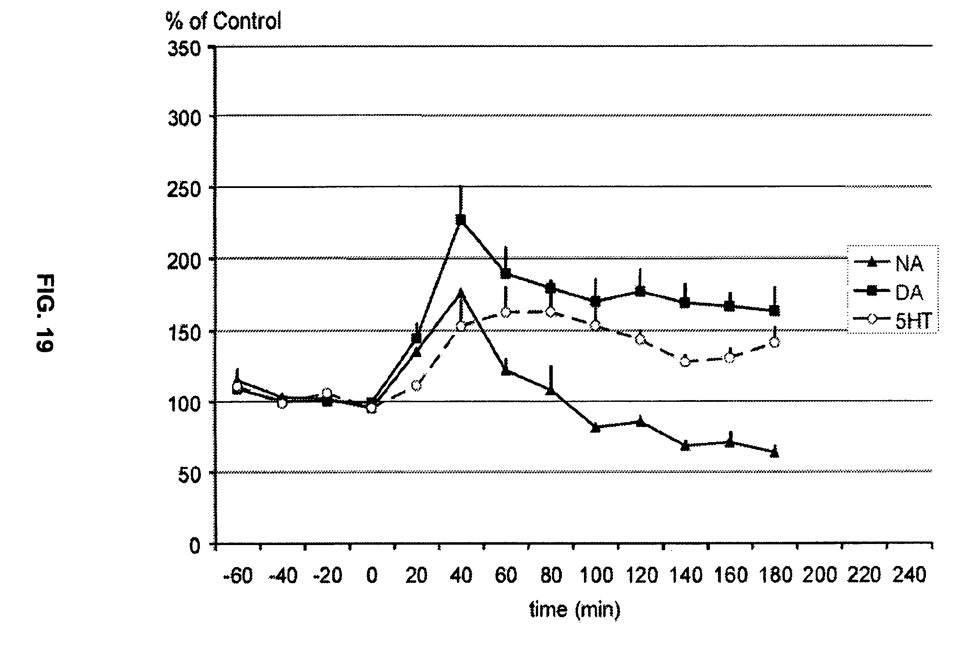

### Figure 19. 4-(3-fluoro-5-trifluoromethyl-phenyl)-1-ethyl-piperidine claimed in WO 01/46146) 50 µmol/kg s.c. striatum amines

4-(3-fluoro-5-trifluoromethyl-phenyl)-1-ethyl-piperidine is injected (s.c) at time-point 0. The values depicted in Figure 19 represent percent of control in relation to baseline values. The microdialysis was performed in awake and freely moving rats. Dopamine = DA; Norepinephrine = NA; Serotonin = 5-HT; Error-bars=SEM

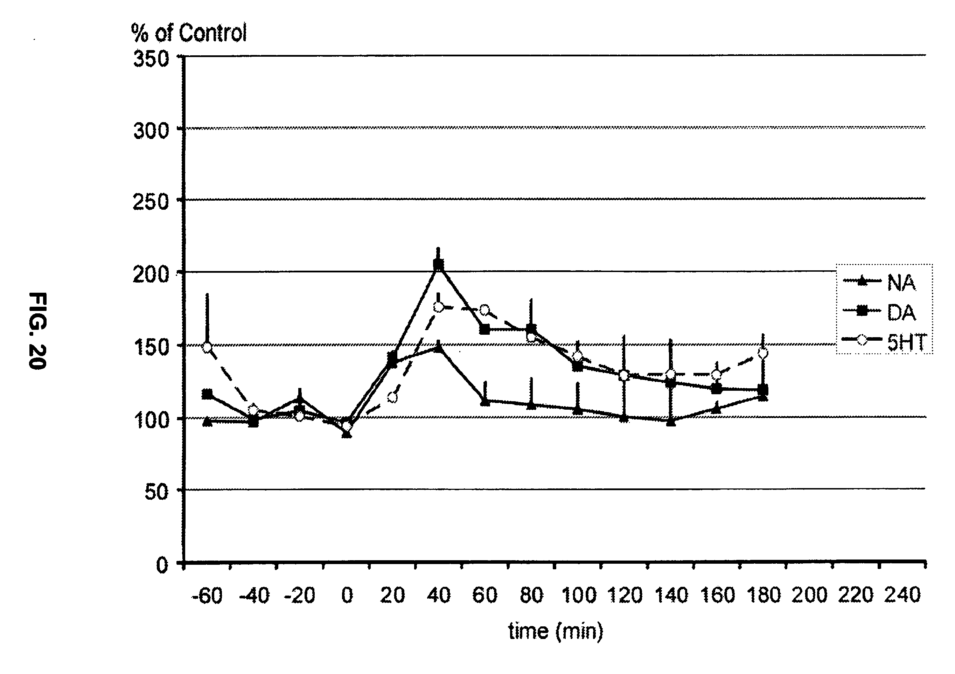

### Figure 20. 4-(3-fluoro-5-trifluoromethyl-phenyl)-1-ethyl-piperidine (claimed in WO 01/46146) 50 µmol/kg s.c.. p.f. cortex

4-(3-fluoro-5-trifluoromethyl-phenyl)-1-ethyl-piperidine is injected (s.c) at time-point 0. The values depicted in Figure 20 represent percent of control in relation to baseline values. The microdialysis was performed in awake and freely moving rats. Dopamine = DA; Norepinephrine = NA; Serotonin = 5-HT; Error-bars=SEM

### Figure 21. Mirtazapine (remeron) 10mg/kg s.c. p.f. striatum

Remeron is injected (s.c.) at time-point 0. The values depicted in Figure 21 represent percent of control in relation to baseline values. The microdialysis was performed in awake and freely moving rats. Dopamine = DA; Norepinephrine = NA; Serotonin = 5-HT; Error-bars=SEM

### Figure 22. Mirtazapine (Remeron) 10mg/kg s.c. p.f. cortex

Remeron is injected (s.c.) at time-point 0. The values depicted in Figure 22 represent percent of control in relation to baseline values. The microdialysis was performed in awake and freely moving rats. Dopamine = DA; Norepinephrine = NA; Serotonin = 5-HT; Error-bars=SEM

### References:

Arnsten A.F.T. and Li B. (2005) Neurobiology of executive functions: Cathecholamine influences on prefrontal cortical functions BIOL PSYCHIATRY 2005;57:1377-1384 Biederman, J. Attention-Deficit/Hyperactivity Disorder: A selective overview BIOL PSYCHIATRY 2005;57:1215-1220.
Harrison, P.J. and Weinberger, D.R. (2005) Schizophrenia genes, gene expression and neuropathology. on the matter of their convergence. Molecular Psychiatry 10: 40-68.
Moghaddam, B. and Bunney, B. S. (1990) Acute effects of typical and atypical antipsychotic drugs on the release of dopamine from prefrontal cortex, nucleus accumbens, and striatum of the rat: an in vivo microdialysis study. J. Neurochem 54, 5:1755-1759.
Deutch AY, Moghaddam B, Innis RB, Krystal JH, Aghajanian GK, Bunney BS, Charney DS. (1991) Mechanisms of action of atypical antipsychotic drugs. Implications for novel therapeutic strategies for schizophrenia. Schizophr Res. Mar-Apr;4(2):121-56.
Pliszka, S.R. (2005) The neuropsychopharmacology of attention-deficit/hyperactivity disorder. Biol Psychiatry. 2005 Jun 1;57(11):1385-90. Review.
Ungerstedt, U. (1991). "Microdialysis-principles and applications for studies in animals and man." J. Int. Med. 230: 365-373.
Ungerstedt, U., M. Herrera-Marschitz, U. Jungnelius, L. Stahle, U. Tossman and T. Zetterström (1982). Dopamine Synaptic Mechanisms Reflected in Studies Combining Behavioural Recordings and Brain Dialysis. Advances in Dopamine Research. M. Kohksa. Oxford, Perganon Press. 37: 219-231.
Devoto, P., G. Flore, L. Pira, G. Longu and G. L. Gessa (2004). "Mirtazapine-induced corelease of dopamine and norepinephrine from noradrenergic neurons in the medial prefrontal and occipital cortex." Eur J Pharmacol 487(1-3): 105-11.
Millan, M. J., F. Lejeune and A. Gobert (2000). "Reciprocal autoreceptor and heteroreceptor control of serotonergic, dopaminergic and noradrenergic transmission in the frontal cortex: relevance to the actions of antidepressant agents." J Psychopharmacol 14(2): 114-38.
Tanda, G., E. Carboni, R. Frau and G. Di Chiara (1994). "Increase of extracellular dopamine in the prefrontal cortex: a trait of drugs with antidepressant potential?" Psychopharmacology (Berl) 115(1-2): 285-8.
Goldman-Rakic, P. et al. (2004) Targeting the dopamine D1 receptor in schizophrenia: insights for cognitive dysfunction. Psychopharmacology 174:3-16.
Arnsten, A. (2004) Adrenergic targets for the treatment of cognitive deficits in schizophrenia. Psychopharmacology 174:25-31.

### METHODS OF PREPARATION

The compounds of the invention may be prepared as outlined below in Scheme 1. However, the invention is not limited to these methods. The compounds may also be prepared as described for structurally-related compounds in the prior art. The reactions can be carried out according to standard procedures (eg. Comprehensive Organic Transformations: A Guide to Functional Group Preparations Richard C. Larock, 22 October, 1999 Wiley-VCH, ISBN: 0471190314; or March's Advanced Organic Chemistry: Reactions, Mechanisms, and Structure, 5th Edition. Michael B. Smith, Jerry March, January 15, 2001 Wiley-Interscience, ISBN: 0471585890) or as described in the working examples. The starting materials for the processes described in the present application are known or may readily be prepared by conventional methods from commercially available chemicals.

Persons skilled in the art will appreciate that, in order to obtain compounds of the invention in an alternative - and in some occasions, more convenient manner - the individual process steps mentioned hereinbefore may be performed in a different order, and/or the individual reactions may be performed at different stage in the overall route (i.e. chemical transformations may be performed upon different intermediates to those associated hereinbefore with a particular reaction).

A synthesis of the compounds of the invention is outlined below in Scheme 1.

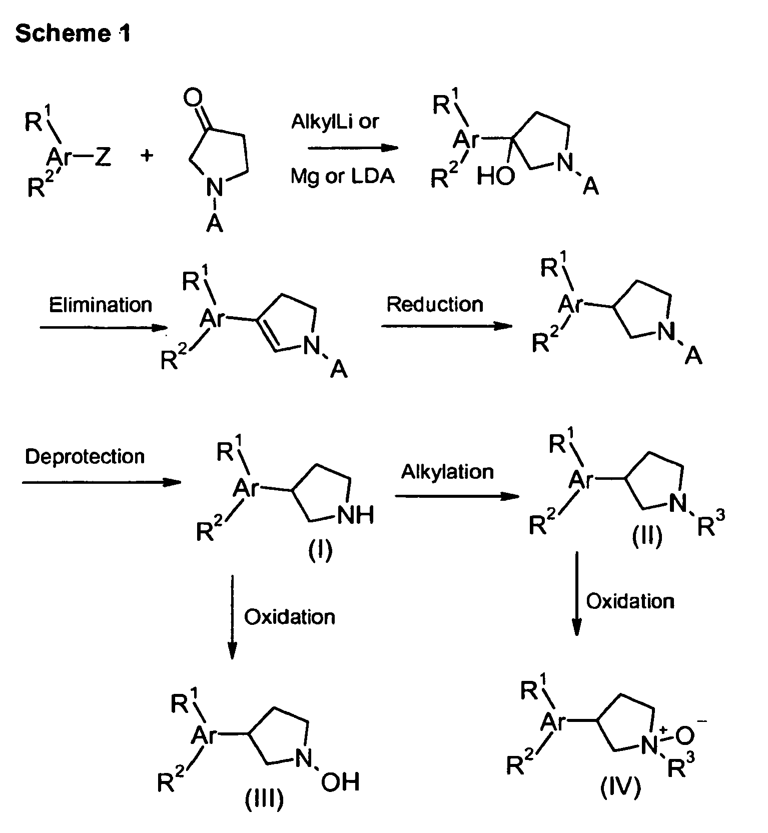

The substituents in Scheme 1 are as follows: Z is a leaving group; A is alkyl or a protecting group; Ar, R1, R2, and R3 are as defined above.

The N-oxide compounds used as prodrugs in the present invention can be synthesised from the amines through standard N-oxidation procedures (e.g. Handbook of Reagents for Organic Synthesis - Oxidising and Reducing Agents. S.D. Burke, R.L. Danheiser (Eds.); John. Wiley & Sons, Chichester, 1999, ISBN 0-471-97926-0)

The compounds of the present invention may be isolated in any level of purity by standard methods and purification can be achieved by conventional means known to those skilled in the art, such as distillation, recrystallization and chromatography.

### Pharmaceutical Compositions

In another aspect the invention provides novel pharmaceutical compositions comprising a therapeutically effective amount of the chemical compound of the invention.

The present invention relates to pharmaceutical compositions comprising the compounds of the present invention, and their use in treating CNS disorders. Both organic and inorganic acids can be employed to form non-toxic pharmaceutically acceptable acid addition salts of the compounds according to the invention. Suitable acid addition salts of the compounds of the present invention include those formed with pharmaceutically acceptable salts such as those mentioned above. The pharmaceutical composition comprising a compound according to the invention may also comprise substances used to facilitate the production of the pharmaceutical preparation or the administration of the preparations. Such substances are well known to people skilled in the art and may for instance be pharmaceutically acceptable adjuvants, carriers and preservatives.

In clinical practice, the compounds according to the present invention will normally be administered orally, rectally, nasally or by injection, in the form of pharmaceutical preparations comprising the active ingredient either as a free base or as a pharmaceutically acceptable non-toxic, acid addition salt, such as the hydrochloride, lactate, acetate or sulfamate salt, in association with a pharmaceutically acceptable carrier. The carrier may be a solid, semisolid or liquid preparation. Usually the active substance will constitute between 0.1 and 99% by weight of the preparation, more specifically between 0.5 and 20% by a weight for preparations intended for injection and between 0.2 and 50% by weight for preparations suitable for oral administration.

To produce pharmaceutical preparations containing the compound according to the invention in the form of dosage units for oral application, the selected compound may be mixed with a solid excipient, e.g. lactose, saccharose, sorbitol, mannitol, starches such as potato starch, corn starch or amylopectin, cellulose derivatives, a binder such as gelatine or polyvinyl-pyrrolidine, and a lubricant such as magnesium stearate, calcium stearate, polyethylene glycol, waxes, paraffin, and the like, and then compressed into tablets. If coated tablets are required, the cores (prepared as described above) may be coated with a concentrated sugar solution which may contain e.g. gum arabic, gelatine, talcum, titanium dioxide, and the like. Alternatively, the tablet can be coated with a polymer known to the man skilled in the art, dissolved in a readily volatile organic solvent or mixture of organic solvents. Dyestuffs may be added to these coatings in order to readily distinguish between tablets containing different active substances or different amounts of the active compound.

For the preparation of soft gelatine capsules, the active substance may be admixed with e.g. a vegetable oil or polyethylene glycol. Hard gelatine capsules may contain granules of the active substance using either the mentioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Examples of tablet and capsule formulations suitable for oral administration are given below:

| **Tablet I** | **mg/tablet** |
|---|---|
| Compound | 100 |
| Lactose Ph.Eur | 182.75 |
| Croscarmellose sodium | 12.0 |
| Maize starch paste (5% w/v paste) | 2.25 |
| Magnesium stearate | 3.0 |

| **Tablet II** | **mg/tablet** |
|---|---|
| Compound | 50 |
| Lactose Ph.Eur | 223.75 |
| Croscarmellose sodium | 6.0 |
| Maize starch | 15.0 |
| Polyvinylpyrrolidone (5% w/v paste) | 2.25 |
| Magnesium stearate | 3.0 |

| **Tablet III** | **mg/tablet** |
|---|---|
| Compound | 1.0 |
| Lactose Ph.Eur | 93.25 |
| Croscarmellose sodium | 4.0 |
| Maize starch paste (5% w/v paste) | 0.75 |
| Magnesium stearate | 1.0 |

| **Capsule** | **mg/capsule** |
|---|---|
| Compound | 10 |
| Lactose Ph.Eur | 488.5 |
| Magnesium | 1.5 |

Dosage units for rectal application can be solutions or suspensions or can be prepared in the form of suppositories comprising the active substance in a mixture with a neutral fatty base, or gelatine rectal capsules comprising the active substance in admixture with vegetable oil or paraffin oil. Liquid preparations for oral application may be in the form of syrups or suspensions, for example solutions containing from about 0.2% to about 20% by weight of the active substance herein described, the balance being sugar and mixture of ethanol, water, glycerol and propylene glycol. Optionally such liquid preparations may contain coloring agents, flavoring agents, saccharine and carboxymethylcellulose as a thickening agent or other excipients known to the man in the art.

Solutions for parenteral applications by injection can be prepared in an aqueous solution of a water-soluble pharmaceutically acceptable salt of the active substance, preferably in a concentration of from 0.5% to about 10% by weight. These solutions may also contain stabilizing agents and/or buffering agents and may conveniently be provided in various dosage unit ampoules. The use and administration to a patient to be treated would be readily apparent to an ordinary skill in the art.

For intranasal administration or administration by inhalation, the compounds of the present invention may be delivered in the form of a solution, dry powder or suspension. Administration may take place via a pump spray container that is squeezed or pumped by the patient or through an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. The compounds of the invention may also be administered via a dry powder inhaler, either as a finely divided powder in combination with a carrier substance (e.g. a saccharide) or as microspheres. The inhaler, pump spray or aerosol spray may be single or multi dose. The dosage may be controlled through a valve that delivers a measured amount of active compound.

The compounds of the invention may also be administered in a controlled release formulation. The compounds are released at the required rate to maintain constant pharmacological activity for a desirable period of time. Such dosage forms provide a supply of a drug to the body during a predetermined period of time and thus maintain drug levels in the therapeutic range for longer periods of time than conventional non-controlled formulations. The compounds may also be formulated in controlled release formulations in which release of the active compound is targeted. For example, release of the compound may be limited to a specific region of the digestive system through the pH sensitivity of the formulation. Such formulations are well known to persons skilled in the art.

Further details on techniques for formulation and administration may be found in the latest edition of Remington's Pharmaceutical Sciences (Maack Publishing Co., Easton, PA).

Depending upon the disorder and patient to be treated and the route of administration, the compositions may be administered at varying doses. The dosing will also depend upon the relation of potency to absorbability and the frequency and route of administration. Such doses may be administered once, twice or three or more times daily. The compounds of this invention can be administered to subjects in doses ranging from 0.01 mg to 500 mg per kg of body weight per day, although variations will necessarily occur depending upon the weight, sex and condition of the subject being treated, the disease state being treated and the particular route of administration chosen. However, a dosage level that is in the range of from 0.1 mg to 10 mg per kg of body weight per day, single or divided dosage is most desirably employed in humans for the treatment of diseases. Alternatively, the dosage level is such that a serum concentration of between 0.1 nM to 10 µM of the compound is obtained.

The invention is further illustrated in the examples below, which in no way are intended to limit the scope of the invention.

### Example 1:

### 3-(3,4-DICHLOROPHENYL)PYRROLIDINE

A mixture of 3-(3,4-dichlorophenyl)-2,5-dihydro-1H-pyrrole (1.0 g, 4.67 mmol) and platinum oxide (0.1 g) in methanol (20 ml) was treated with hydrogen at 50 psi for 15 h. The reaction mixture was filtered through a pad of celite and the filtrate was evaporated. Aqueous sodium carbonate (10%, 50 ml) was added and the aqueous phase was extracted with ethylacetate (3x30 ml). The combined organic phases was dried (Na2SO4), filtered and evaporated.Purification by preparative HPLC followed by flash chromatography on silica gel gave the title compound (0.2 g). The amine was converted to the hydrochloric acid salt and recrystallized from methanol/diethyl ether: M.p. 136-137 °C. MS m/z (relative intensity, 70 eV) 217 (M+, 63), 215 (M+, bp), 172 (18), 151 (37), 115 (60).

### Example 2:

### 3-(2,4-DIFLUOROPHENYL)PYRROLIDINE

A mixture of 1-benzyl-3-(2,4-difluorophenyl)-3-fluoropyrrolidine (0.80 g, 2.75 mmol) palladium on carbon (0.08 g) and ammonium formiate (1.73 g, 27.5 mmol) in methanol (20 ml), was refluxed for 20 minutes. The mixture was filtered through a pad of celite and the filtrate was evaporated. Aqeuous sodium carbonate (10%, 50 ml) was added and the aqueous phase was extracted with ethyl acetate (2x50 ml). The combined organic phase was dried and evaporated to give the title compound. (0.37 g) The amine was converted to the oxalic acid salt and recrystallized from methanol/diethyl ether: M.p. 116-116 °C. MS m/z (relative intensity, 70 eV) 183 (M+, bp), 153 (35), 140 (22), 133 (21), 127 (48).

### Example 3:

### 3-(3,5-DIFLUOROPHENYL)PYRROLIDINE

Preparation according to Example 2: 1-benzyl-3-(3,5-difluorophenyl)-2,5-dihydro-1H-pyrrole (0.71 g, 2.62 mmol), ammonium formiate (1.65 g, 26.2 mmol), palladium on carbon (0.07 g), methanol (50 ml). After filtration and evaporation of methanol, aqeuous sodium carbonate (10%, 50 ml) was added and the aqueous phase was extracted with ethyl acetate (2x50 ml). The combined organic phase was evaporated, aqueous hydrochloric acid (5%, 40 ml) was added, the aqueous phase was washed with two portions of diethyl ether and then basified by the addition of aqeuous sodium carbonate. Ethyl acetate was added and the organic phase was separated, dried (MgSO4) and evaporated.. The amine was converted to the oxalic acid salt and recrystallized from ethanol/diethyl ether: M.p. 199-200 °C. MS m/z (relative intensity, 70 eV) 183 (M+, bp), 153 (44), 151 (24), 133 (23), 127 (43).

### Example 4:

### 3-(3,4-DIFLUOROPHENYL)-1-METHYLPYRROLIDINE

A mixture of 3-(3,4-difluorophenyl)pyrrolidine (0.31 g, 1.69 mmol) in formic acid (4.9 ml) and formaldehyde (40% solution, 4.4 ml) was heated at 100 °C for 1 h. Water (50 ml) was added and the solution was basified by the addition of aqueous sodium hydroxide (5M, 20 ml). The aqueous phase was extracted with ethyl acetate (2x50 ml), the combined organic phases was dried (MgSO4) and evaporated under reduced pressure to give the crude product (0.33 g). Purification by flash chromatography (ethyl acetate/methanol 1:1) yields 0.19 g (57%) of the title compound. The amine was converted to the fumaric acid salt and recrystallized from ethanol/diisopropyl ether: M.p. 140-142 °C. MS ml) (relative intensity, 70 eV) 197 (M+, 71), 196 (23), 153 (13), 127 (19), 57 (bp).

### Example 5:

### 3-(3,4-DIFLUOROPHENYL)PYRROLIDINE

A mixture of 1-benzyl-3-(3,4-difluorophenyl)-2,5-dihydro-1H-pyrrole (1.96 g, 7.23 mmol) and ammonium formiate (4.55 g, 72.3 mmol) in methanol (20 ml) was purged with nitrogen gas after which palladium on carbon (0.2 g) was added. The mixture was refluxed for 2 h, cooled to ambient temperature and filtered through a pad of celite. The filtrate was evaporated and the crude product was purified by preparative HPLC to give the title compound (0.3 g). The amine was converted to the hydrochloric acid salt and recrystallized from ethanol/diethyl ether: M.p. 139-140 °C. MS m/z (relative intensity, 70 eV) 183 (M+, bp), 153 (38), 151 (22), 133 (24), 127 (45).

The following Preparations are used in the synthesis of the above Examples.

### Preparation 1:

### 1-BENZYL-3-(3,5-DIFLUOROPHENYL)PYRROLIDIN-3-OL

To a solution of 1-bromo-3,5-difluorobenzene (2.5 g, 12.8 mmol) in dry diethyl ether (30 ml), under nitrogen, was added dropwise at -78°C, hexyllithium (2.3 M in hexane, 5.6 ml, 12.8 mmol). The mixture was stirred for 1 minute after which a solution of 1-benzylpyrrolidin-3-one (1.5 g, 8.6 mmol) in dry diethyl ether (20 ml) was added drop wise. The resulting mixture was brought to ambient temperature, water (20 ml) was added and the mixture was extracted with ethylacetate (3x50 ml). The combined organic phase was washed with brine, dried (MgSO4), filtered and evaporated. Purification by flash column chromatography (ethylacetate/isooctane, 1:1) gave the title compound (2.07 g). MS m/z (rel. intensity, 70 eV) 289 (M+, 7), 198 (60),134 (34), 133 (56), 132 (43), 91 (bp).

### Preparation 2:

### 1-BENZYL-3-(3,5-DIFLUOROPHENYL)-2,5-DIHYDRO-1H-PYRROLE

A mixture of 1-benzyl-3-(3,5-difluorophenyl)pyrrolidin-3-ol (1.6 g, 5.5 mmol) and polyphosphoric acid (2 g) was heated at 85 °C for 1.5 h. The mixture was cooled to ambient temperature, water (50 ml) was added and the mixture was basified with aqueous sodium hydroxide (5 M). Dichloromethane was added and the organic phase was dried (MgSO4) and evaporated. Purification by column chromatography on silica gel gave the title compound (0.71 g). MS m/z (rel. intensity, 70 eV) 271 (M+, bp), 270 (56), 369 (93), 180 (26), 91 (93).

### Preparation 3:

### 1-BENZYL-3-(3,4-DIFLUOROPHENYL)PYRROLIDIN-3-OL

To a solution of 1-bromo-3,4-difluorobenzene (3.0 g, 15.5 mmol) in dry diethyl ether (25 ml), under nitrogen, was added dropwise at -78 °C, n-butyllithium (2.5 M in hexane, 6.25 ml, 15.5 mmol). The mixture was stirred for 1 h after which a solution of 1-benzylpyrrolidin-3-one (2.7 g, 15.5 mmol) in dry diethyl ether (25 ml) was added drop wise. The resulting mixture was brought to ambient temperature, stirred for 0.5 h, water (20 ml) was added and the mixture was extracted with ethylacetate (2x50 ml). The combined organic phase was dried (Na2SO4), filtered and evaporated.. Purification by flash column chromatography on silica gel (ethylacetate/isooctane, 1:1) gave the title compound (2.7 g). MS m/z (rel. intensity, 70 eV) 289 (M+, 9), 198 (bp), 134 (23), 133 (35), 132 (30), 91 (63).

### Preparation 4:

### 1-BENZYL-3-(3,4-DIFLUOROPHENYL)-2,5 AND 2,3 -DIHYDRO-1H-PYRROLE

1-benzyl-3-(3,4-difluorophenyl)pyrrolidin-3-ol (1.6 g, 5.5 mmol) and trifluoroacetic acid (20 ml) was heated at 60 °C for 14 h and at 90 °C for 4 h. The trifluoroacetic acid was evaporated, aqeuous sodium carbonate (10%, 50 ml) was added and the aqueous phase was extracted with ethyl acetate (50 ml). Water (50 ml) was added and the solution was basified with aqueous sodium hydroxide (5 M). The combined organic phase was evaporated, aqueous hydrochloric acid (5%, 40 ml) was added, the aqueous phase was washed with two portions of diethyl ether and then basified by the addition of aqeuous sodium carbonate. Ethyl acetate was added and the organic phase was separated, dried (MgSO4) and evaporated to give the title compounds. (1.96 g) MS m/z (relative intensity, 70 eV) 271 (M+, 51), 270 (31), 269 (31), 180 (35), 91 (bp).

### Preparation 5:

### 1-BENZYL-3-(2,4-DIFLUOROPHENYL)PYRROLIDIN-3-OL

Preparation according to Preparation 1: 1-bromo-2,4-difluorobenzene (7.49 g, 38.5 mmol), dry diethyl ether (60 ml), hexyllithium (2.3 M in hexane, 16.8 ml, 38.5 mmol) and 1-benzylpyrrolidin-3-one (4.5 g, 25,7 mmol). Yield: 5.7 g. MS m/z (rel. intensity, 70 eV) 289 (M+, 5), 198 (66), 133 (52), 132 (42), 91 (bp).

### Preparation 6:

### 1-BENZYL-3-(2,4-DIFLUOROPHENYL)-3-FLUOROPYRROLIDINE

To a cooled (0 °C) solution of 1-benzyl-3-(2,4-difluorophenyl)pyrrolidin-3-ol (1.9 g, 6.57 mmol) in dichloromethane (50 ml) was added dropwise a solution of diethylaminosulphurtrifluoride (0.86 ml, 6.57 mmol) in dichloromethane (25 ml). The mixture was stirred for 0.5 h after which aqeuous sodium carbonate (50 ml) was added and the phases separated. The aqueous phase was extracted with dichloromethane (50 ml) and the combined organic phase was dried (MgSO4) and evaporated.Purification by flash column chromatography on silica gel gave the title compound (0.8 g). MS m/z (rel. intensity, 70 eV) 291 (M+, 59), 271 35), 133 (64), 132 (49), 91 (bp).

### Preparation 7:

### TERT-BUTYL 3-(3,4-DICHLOROPHENYL)-3-HYDROXYPYRROLIDINE-1-CARBOXYLATE

To a solution of 4-bromo-1,2-dichlorobenzene (2.0 g, 8.85 mmol) in dry tetrahydrofurane (35 ml), under nitrogen, was added magnesium turnings (0.21 g, 8,85 mmol). The mixture was refluxed for 1 h, cooled to ambient temperature and a solution of 1-N-boc-3-pyrrolidone (1.63 g, 8.85 mmol) in dry tetrahydrofurane (10 ml) was added drop wise. The resulting mixture was refluxed for 3 h after which aqueous saturated ammonium chloride solution (40 ml) was added and the mixture was extracted with ethylacetate (3x50 ml). The combined organic phase was dried (MgSO4), filtered and evaporated. Purification by flash chromatography on silica gel (isooctane/ethyl acetate 1:1) gave the title compound (1 g). MS m/z (rel. intensity, 70 eV) 332 (M+, 1), 275 (41), 232 (37), 231 (28), 230 (52), 57 (bp).

### Preparation 8:

### 3-(3,4-DICHLOROPHENYL)-2,5-DIHYDRO-1H-PYRROLE

tert-Butyl 3-(3,4-dichlorophenyl)-3-hydroxypyrrolidine-1-carboxylate (6.15 g, 18.5 mmol) and trifluoroacetic acid (20 ml) was heated at 70 °C for 14 h. The mixture was cooled to 0 °C and aqeuous sodium hydroxide (5N) was added until pH reached 10. The aqueous phase was extracted with ethyl acetate (2x50 ml) dried (Na2SO4) and evaporated.. Purification by flash column chromatography (Methanol) gave the title compound (1. g). MS m/z (relative intensity, 70 eV) 215 (45), 214 (M+, 72), 213 (76), 212 (bp), 177 (68).

The following tests were used for evaluation of the compounds according to the invention.

### In vivo test: Neurochemistry

Male Sprague-Dawley rats weighing 220-320g are used throughout the experiments. Sixty (60) minutes following administration of the test substance, the rats are decapitated. Directly after decapitation the brain is removed from the skull and put on a glass petri bowl filled with ice. The limbic system (containing the nucleus accumbens - both the core and shell, most parts of the olfactory tubercle and ventral pallidum) is dissected using two thin, angled tweezers and put directly on foil on dry ice (carbon dioxide -78° C). The striatum and cortex are then dissected and also put on dry ice. The time from decapitation until the last tissue is dissected varies from four to six minutes.. The tissue is weighed using a Sartorius BP3105 connected to a computer and packed in labelled tin foil, then stored in an -80° C freezer. Great care is taken in order to keep the tissue frozen until the time of neurochemical analysis. Each brain part is subsequently analyzed with respect to its content of monoamines and their metabolites.

The monoamine transmitter substances (NE (norepinephrine), DA (dopamine), 5-HT (serotonin)) as well as their amine (NM (normethanephrine), 3-MT (3-methoxytyramine)) and acid (DOPAC (3,4-dihydroxyphenylacetic acid), 5-HIAA (5-hydroxy-indoleacetic acid), HVA (homovanillic acid)) metabolites are quantified in brain tissue homogenates by HPLC separations and electrochemical detection.

The analytical method is based on two chromatographic separations dedicated for amines or acids. Two chromatographic systems share a common auto injector with a 10-port valve and two sample loops for simultaneous injection on the two systems. Both systems are equipped with a reverse phase column (Luna C18(2), dp 3 µm, 50*2mm i.d., Phenomenex) and electrochemical detection is accomplished at two potentials on glassy carbon electrodes (MF-1000, Bioanalytical Systems, Inc.). The column effluent is passed *via* a T-connection to the detection cell or to a waste outlet. This is accomplished by two solenoid valves, which block either the waste or detector outlet. By preventing the chromatographic front from reaching the detector, better detection conditions are achieved. The aqueous mobile phase (0.4 ml/min) for the acid system contains citric acid 14 mM, sodium citrate 10 mM, MeOH 15% (v/v) and EDTA 0.1 mM. Detection potentials relative to Ag/AgCl reference are 0.45 and 0.60V. The aqueous ion pairing mobile phase (0.5 ml/min) for the amine system contains citric acid 5 mM, sodium citrate 10 mM, MeOH 9% (v/v), MeCN 10.5% (v/v), decane sulfonic acid 0.45 mM, and EDTA 0.1 mM. Detection potentials relative to Ag/AgCl reference are 0.45 and 0.65V.

### In vivo test: Microdialysis

Male Sprague-Dawley rats weighing 220-320g were used throughout the experiments. Before the experiment the animals were group housed, five animals in each cage, with free access to water and food. The animals were housed at least 5 days after arrival prior to surgery and use in the experiments. Each rat was used only once for microdialysis.

We use a modified version (Waters, Lofberg et al. 1994) of the I-shaped probe (Santiago and Westerink 1990). The dialysis membrane we use is the AN69 polyacrylonitrile/ sodium methalylsulfonate copolymer (HOSPAL; o.d./i.d. 310/220 µm: Gambro, Lund, Sweden). In the dorsal striatum we use probes with an exposed length of 3 mm of dialysis membrane and in the prefrontal cortex the corresponding length is 2.5 mm. The rats were operated under isoflurane inhalation anesthesia while mounted into a Kopf stereotaxic instrument. Coordinates were calculated relative to bregma; dorsal striatum AP +1, ML ± 2.6, DV -6.2; Pf cortex, AP +3.2, 8° ML ±1.2, DV -4.0 according to (Paxinos and Watson 1986). The dialysis probe was positioned in a burr hole under stereotaxic guidance and cemented with phosphatine dental cement.

The rats were housed individually in cages for 40 h before the dialysis experiments, allowing them to recover from surgery and minimizing the risk of drug interactions with the anaesthetic during the following experiments. During this period the rats had free access to food and water. On the day of experiment the rats were connected to a micro perfusion pump via a swivel and were replaced in the cage where they could move freely within its confinements. The perfusion medium was a Ringer's solution containing in mmol/l: NaCl; 140, CaCl2; 1.2, KCl; 3.0, MgCl2; 1.0 and ascorbic acid; 0.04 according to (Moghaddam and Bunney 1989). The pump was set to a perfusion speed of 2 µl/min and 40 µl samples were collected every 20 min.

The monoamine transmitter substances (NE (norepinephrine), DA (dopamine), 5-HT (serotonin)) as well as their amine (NM (normethanephrine), 3-MT (3-methoxytyramine)) and acid (DOPAC (3,4-dihydroxyphenylacetic acid), 5-HIAA (5-hydroxy-indoleacetic acid), HVA (homovanillic acid)) metabolites are quantified in brain tissue homogenates by HPLC separations and electrochemical detection.

The monoamine transmitter substances (NA, DA, 5-HT) as well as their amine (NM, 3-MT) and acid (DOPAC, 5-HIAA, HVA) metabolites are quantified in micro dialysis samples by HPLC separations and electrochemical detection.

The analytical method is based on two chromatographic separations dedicated for amines or acids. Two chromatographic systems share a common auto-injector with a 10-port valve and two sample loops (5 µl for acids, 20 µl for amines) for simultaneously injection on the two systems. The acids are separated by reverse phase chromategraphy while the amines are separated by reverse phase ion pairing preceded by a reverse phase separation in a column switching configuration.

Three separation columns (Luna C18(2), dp 3 µm, 2mm i.d., Phenomenex) of different lengths are used. Electrochemical detection is accomplished on glassy carbon electrodes (MF-1000, Bioanalytical Systems, Inc.).

The aqueous mobile phase (0.6 ml/min) for the acid system contains Citric Acid 40 mM, di-Potassium hydrogen phosphate 10 mM, MeOH 8% (v/v) and EDTA 0.1 mM. Column length is 30mm. Detection potential relative to Ag/AgCl reference is 0.70V.

The aqueous ion pairing mobile phase (0.4 ml/min) for the amine system contains Citric Acid 5 mM, Sodium Citrate 10 mM, MeCN 10%(v/v), THF 4%(v/v), Dodecane Sulfonic Acid 0.05 mM, and EDTA 0.1 mM. Column length is 50mm. Detection potentials relative to Ag/AgCl reference are 0.45 and 0.65V.

The aqueous mobile phase (0.4 ml/min) for the coupled reverse phase separation is identical to the ion pairing mobile phase, but Dodecane Sulfonic Acid is excluded. Column length is 20mm. Minor modifications in analytical conditions may occur over time for optimisation.

After the experiment the rats were uncoupled from the perfusion pump and decapitated. Their brains were rapidly taken out and fixed in Accustain solution (Sigma, Sweden) for subsequent inspection of probe localisation. The Animal Ethics Committee in Göteborg, Sweden approved the procedures applied in these experiments.

For comparative example 16 of reference 1 an earlier analytical procedure was used. In this procedure the amines are separated without column switching and the ion pairing conditions are slightly different optimised. For comparative example 16 in reference 1, anesthesia was induced by injection of ketamine and xylazine, and the brains were fixed in Neo-fix solution (Kebolab, Sweden) for subsequent inspection of probe localisation.

### References:

Moghaddam, B. and B. S. Bunney (1989). "Ionic Composition of Microdialysis Perfusing Solution Alters the Pharmacological Responsiveness and Basal Outflow of Striatal Dopamine." J. Neurochem. 53: 652-654.
Paxinos, G. and C. Watson (1986). The Rat Brain in Stereotaxic Coordinates. New York, Academic Press.
Santiago, M. and B. H. C. Westerink (1990). "Characterization of the in vivo release of dopamine as recorded by different types of intracerebral microdialysis probes." Naunyn-Schmiedeberg's Arch. Pharmacol. 342: 407-414.
Waters, N., L. Lofberg, S. Haadsma-Svensson, K. Svensson, C. Sonesson and A. Carlsson (1994). "Differential effects of dopamine D2 and D3 receptor antagonists in regard to dopamine release, in vivo receptor displacement and behaviour." J Neural Transm Gen Sect 98(1): 39-55.

## Claims

1. A compound of Formula (2): 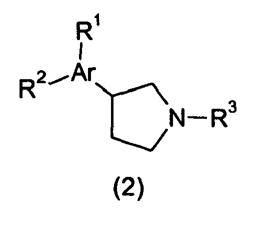
any of its stereoisomers or any mixture of its stereoisomers, or an N-oxide thereof, or a pharmaceutically acceptable salt thereof, wherein:
Ar is phenyl;
R¹ is selected from the group consisting of F and Cl;
R² is selected from the group consisting of F and Cl; and
R³ is selected from the group consisting of H and Me;
with the proviso that when one of R¹ and R² is located in the para-position and
the other of R¹ and R² is located in the meta-position, then R¹ and R² are not both F when R³ is H.

2. A compound according to claim 1, of Formula (3): 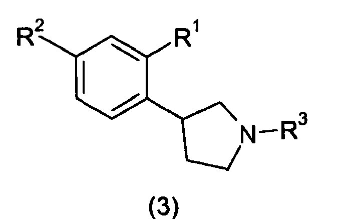 or Formula (4): 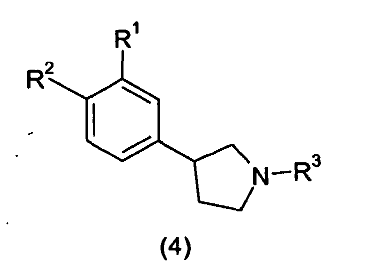 or Formula (5): 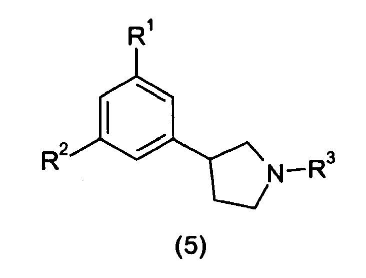 or Formula (6): 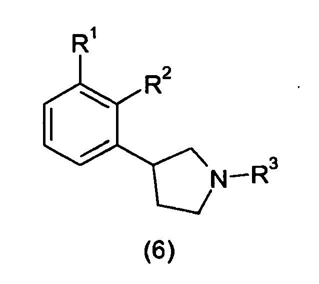 wherein R¹, R² and R³ are as defined in claim 1;
with the proviso that, in Formula (5) above, R¹ and R² are not both F when R³ is H.

3. A compound according to either one of claims 1-2, wherein R¹ is F.

4. A compound according to any one of claims 1-3, wherein R² is F when R³ is H or Me.

5. A compound according to any one of claims 1-4, wherein R³ is H.

6. A compound according to any one of claims 1-5, in the (+)-enantiomeric form.

7. A compound according to any one of claims 1-5, in the (-)-enantiomeric form.

8. The compound according to claim 1, which is
3-(3,4-DICHLOROPHENYL)PYRROLIDINE;
3-(2,4-DIFLUOROPHENYL)PYRROLIDINE;
3-(3,5-DIFLUOROPHENYL)PYRROLIDINE; or
3-(3,4-DIFLUOROPHENYL)-1-METHYLPYRROLIDINE;
any of its stereoisomers or any mixture of its stereoisomers,
or an N-oxide thereof, or a pharmaceutically acceptable salt thereof.

9. A pharmaceutical composition comprising a therapeutically effective amount of a compound according to any one of claims 1-8; or the compound 3-(3,4-DIFLUOROPHENYL)PYRROLIDINE:
any of its stereoisomers or any mixture of its stereoisomers, or an N-oxide thereof, or a pharmaceutically acceptable salt thereof, together with one or more pharmaceutically acceptable carriers or diluents.

10. Use of the compound according to any one of claims 1-8; or the compound 3-(3,4-DIFLUOROPHENYL)PYRROLIDINE;
any of its stereoisomers or any mixture of its stereoisomers, or an N-oxide thereof, or a pharmaceutically acceptable salt thereof, for the manufacture of a medicament.

11. The use according to claim 10, for the manufacture of a pharmaceutical composition for the treatment, prevention or alleviation of a disease or a central nervous system disorder of a mammal, including a human.

12. The use according to claim 11, wherein the central nervous system disorder is a cognitive disorder, a neurodegenerative disorder, dementia, age-related cognitive impairment, a developmental disorder, an Autism spectrum disorder, ADHD, Cerebral Palsy, Gilles de la Tourette's syndrome, a cognitive disorder occurring as part of the core symptoms of schizophrenia, schizophrenia, a schizophreniform disorder, an affective disorder, depression, bipolar disorder, a anxiety disorder, generalized anxiety disorder (GAD), specific phobia, panic disorder (PD), or a sleep disorder.

13. A compound according to any one of claims 1-8; or the compound 3-(3,4-DIFLUOROPHENYL)PYRROLIDINE;
any of its stereoisomers or any mixture of its stereoisomers, or an N-oxide thereof, or a pharmaceutically acceptable salt thereof, for use as a medicament.

14. A compound according to any one of claims 1-8; or the compound 3-(3,4-DIFLUOROPHENYL)PYRROLIDINE;
any of its stereoisomers or any mixture of its stereoisomers, or an N-oxide thereof, or a pharmaceutically acceptable salt thereof, for use in the treatment, prevention or alleviation of a disease or a disorder or a condition of a mammal, including a human, which disease, disorder or condition is responsive to modulation of dopaminergic function in the central nervous system.

## Patentansprüche

1. Verbindung der Formel (2): 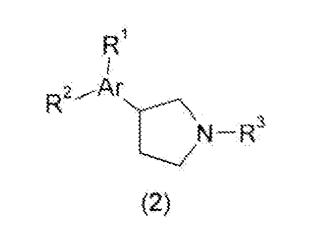 beliebige von ihren Stereoisomeren oder eine beliebige Mischung von ihren Stereoisomeren, oder ein N-Oxid davon, oder ein pharmazeutisch verträgliches Salz davon, wobei:
Ar Phenyl ist;
R¹ ausgewählt ist aus der Gruppe bestehend aus F und Cl;
R² ausgewählt ist aus der Gruppe bestehend aus F und Cl; und
R³ ausgewählt ist aus der Gruppe bestehend aus H und Me;
mit der Maßgabe, dass, wenn eines von R¹ und R² sich in der para-Stellung befindet und
das andere von R¹ und R² sich in der meta-Stellung befindet, dann R¹ und R² nicht beide F sind, wenn R³ H ist.

2. Verbindung nach Anspruch 1, der Formel (3): 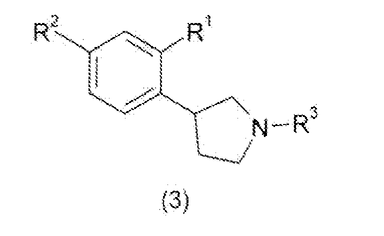 oder der Formel (4): 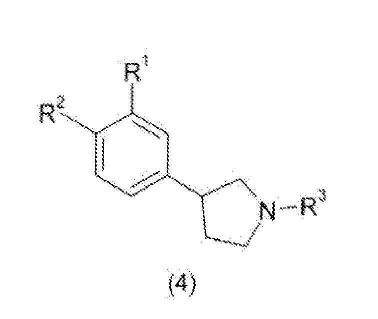 oder der Formel (5): 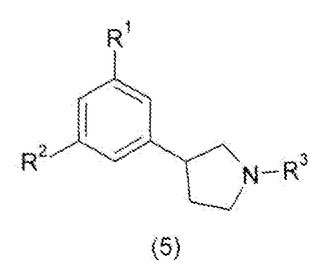 oder der Formel (6): 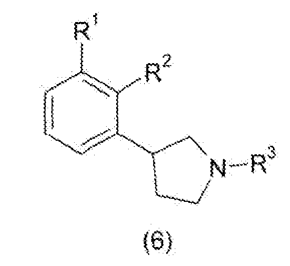 wobei R¹, R² und R³ wie in Anspruch 1 definiert sind;
mit der Maßgabe, dass in vorstehender Formel (5) R¹ und R² nicht beide F sind, wenn R³ H ist.

3. Verbindung nach einem der Ansprüche 1-2, wobei R¹ F ist.

4. Verbindung nach einem der Ansprüche 1-3, wobei R² F ist, wenn R³ H oder Me ist.

5. Verbindung nach einem der Ansprüche 1-4, wobei R³ H ist.

6. Verbindung nach einem der Ansprüche 1-5, in der (+)-enantiomeren Form.

7. Verbindung nach einem der Ansprüche 1-5, in der (-)-enantiomeren Form.

8. Verbindung nach Anspruch 1, welche
3-(3,4-Dichlorphenyl)pyrrolidin;
3-(2,4-Difluorphenyl)pyrrolidin;
3-(3,5-Difluorphenyl)pyrrolidin; oder
3-(3,4-Difluorphenyl)-1-methylpyrrolidin ist;
beliebige von ihren Stereoisomeren oder eine beliebige Mischung von ihren Stereoisomeren, oder ein N-Oxid davon, oder ein pharmazeutisch verträgliches Salz davon.

9. Pharmazeutische Zusammensetzung umfassend eine therapeutisch wirksame Menge von einer Verbindung nach einem der Ansprüche 1-8; oder der Verbindung 3-(3,4-Difluorphenyl)pyrrolidin; beliebigen von ihren Stereoisomeren oder einer beliebigen Mischung von ihren Stereoisomeren, oder einem N-Oxid davon, oder einem pharmazeutisch verträglichen Salz davon, zusammen mit einem oder mehreren pharmazeutisch verträglichen Trägern oder Verdünnungsmitteln.

10. Verwendung der Verbindung nach einem der Ansprüche 1-8; oder der Verbindung 3-(3,4-Difluorphenyl)pyrrolidin; von beliebigen von ihren Stereoisomeren oder einer beliebigen Mischung von ihren Stereoisomeren, oder einem N-Oxid davon, oder einem pharmazeutisch verträglichen Salz davon, für die Herstellung eines Medikaments.

11. Verwendung nach Anspruch 10, für die Herstellung einer pharmazeutischen Zusammensetzung für die Behandlung, Verhütung oder Linderung einer Krankheit oder einer Störung des Zentralnervensystems eines Säugers, einschließlich eines Menschen.

12. Verwendung nach Anspruch 11, wobei die Störung des Zentralnervensystems eine kognitive Störung, eine neurodegenerative Störung, Demenz, altersbedingte kognitive Beeinträchtigung, eine Entwicklungsstörung, eine Autismusspektrumstörung, ADHS (Aufmerksamkeitsdefizit-/Hyperaktivitätsstörung), Cerebralparese, das Gilles de la Tourette-Syndrom, eine kognitive Störung, die als Teil der Kernsymptome von Schizophrenie auftritt, Schizophrenie, eine schizophreniforme Störung, eine affektive Psychose, eine Depression, eine bipolare Störung, eine Angststörung, eine generalisierte Angststörung (GAS), eine spezifische Phobie, eine Panikstörung (PS) oder eine Schlafstörung ist.

13. Verbindung nach einem der Ansprüche 1-8; oder die Verbindung 3-(3,4-Difluorphenyl)pyrrolidin; beliebige von ihren Stereoisomeren oder eine beliebige Mischung von ihren Stereoisomeren, oder ein N-Oxid davon, oder ein pharmazeutisch verträgliches Salz davon, zur Verwendung als ein Medikament.

14. Verbindung nach einem der Ansprüche 1-8; oder die Verbindung 3-(3,4-Difluorphenyl)pyrrolidin; beliebige von ihren Stereoisomeren oder eine beliebige Mischung von ihren Stereoisomeren, oder ein N-Oxid davon, oder ein pharmazeutisch verträgliches Salz davon, zur Verwendung bei der Behandlung, Verhütung oder Linderung einer Krankheit oder einer Störung oder eines Zustands eines Säugers, einschließlich eines Menschen, wobei die Krankheit, die Störung oder der Zustand auf die Modulation der dopaminergen Funktion im Zentralnervensystem anspricht.

## Revendications

1. Composé de formule (2) : 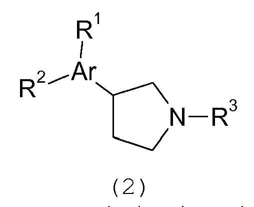 l'un quelconque de ses stéréo-isomères ou tout mélange de ses stéréo-isomères, ou un N-oxyde de celui-ci, ou un sel pharmaceutiquement acceptable de celui-ci, dans laquelle :
Ar est un phényle ;
R¹ est choisi dans le groupe constitué par F et Cl ;
R² est choisi dans le groupe constitué par F et Cl ; et
R³ est choisi dans le groupe constitué par H et Me ;
à la condition que, lorsque l'un parmi R¹ et R² se trouve en position para et l'autre parmi R¹ et R² se trouve en position méta, alors R¹ et R² ne soient pas tous les deux F lorsque R³ est H.

2. Composé selon la revendication 1, de formule (3) : 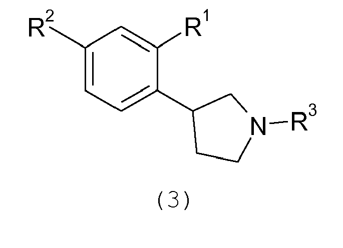 ou de formule (4) : 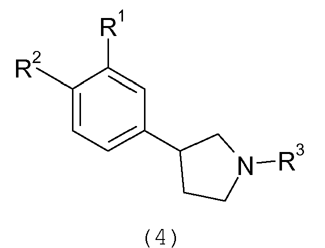 ou de formule (5) : 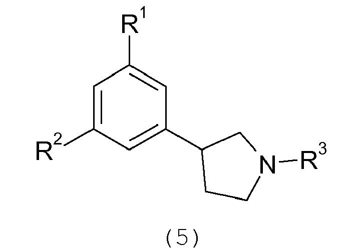 ou de formule (6) : 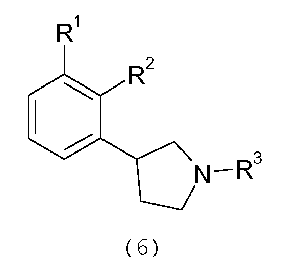 dans laquelle R¹, R² et R³ sont tels que définis dans la revendication 1 ;
à la condition que, dans la formule (5) ci-dessus, R¹ et R² ne soient pas tous les deux F lorsque R³ est H.

3. Composé selon l'une quelconque des revendications 1 et 2, dans lequel R¹ est F.

4. Composé selon l'une quelconque des revendications 1 à 3, dans lequel R² est F lorsque R³ est H ou Me.

5. Composé selon l'une quelconque des revendications 1 à 4, dans lequel R³ est H.

6. Composé selon l'une quelconque des revendications 1 à 5, sous la forme énantiomère (+).

7. Composé selon l'une quelconque des revendications 1 à 5, sous la forme énantiomère (-).

8. Composé selon la revendication 1, qui est
la 3-(3,4-dichlorophényl)pyrrolidine ;
la 3-(2,4-difluorophényl)pyrrolidine ;
la 3-(3,5-difluorophényl)pyrrolidine ; ou
la 3-(3,4-difluorophényl)-1-méthylpyrrolidine ;
l'un quelconque de ses stéréo-isomères ou tout mélange de ses stéréo-isomères, ou un N-oxyde de celui-ci ou un sel pharmaceutiquement acceptable de celui-ci.

9. Composition pharmaceutique comprenant une quantité thérapeutiquement efficace d'un composé selon l'une quelconque des revendications 1 à 8 ; ou le composé 3-(3,4-difluorophényl)pyrrolidine ;
l'un quelconque de ses stéréo-isomères ou tout mélange de ses stéréo-isomères, ou un N-oxyde de celui-ci ou un sel pharmaceutiquement acceptable de celui-ci, conjointement avec un ou plusieurs supports ou diluants pharmaceutiquement acceptables.

10. Utilisation du composé selon l'une quelconque des revendications 1 à 8 ; ou du composé 3-(3,4-difluorophényl)pyrrolidine ;
de l'un quelconque de ses stéréo-isomères ou de tout mélange de ses stéréo-isomères, ou d'un N-oxyde de celui-ci ou d'un sel pharmaceutiquement acceptable de celui-ci, pour la fabrication d'un médicament.

11. Utilisation selon la revendication 10, pour la fabrication d'une composition pharmaceutique pour le traitement, la prévention ou l'atténuation d'une maladie ou d'un trouble du système nerveux central d'un mammifère, incluant un être humain.

12. Utilisation selon la revendication 11, dans laquelle le trouble du système nerveux central est un trouble cognitif, un trouble neurodégénératif, une démence, une détérioration cognitive liée à l'âge, un trouble du développement, un trouble du spectre de l'autisme, un THADA, une infirmité motrice cérébrale, un syndrome de Gilles de la Tourette, un trouble cognitif se produisant en tant que partie des symptômes majeurs d'une schizophrénie, une schizophrénie, une trouble schizophréniforme, un trouble affectif, une dépression, un trouble bipolaire, un trouble d'anxiété, un trouble d'anxiété généralisée (TAG), une phobie spécifique, un trouble panique (TP) ou un trouble du sommeil.

13. Composé selon l'une quelconque des revendications 1 à 8 ; ou le composé 3-(3,4-difluorophényl)pyrrolidine ;
l'un quelconque de ses stéréo-isomères ou tout mélange de ses stéréo-isomères, ou un N-oxyde de celui-ci ou un sel pharmaceutiquement acceptable de celui-ci, pour une utilisation en tant que médicament.

14. Composé selon l'une quelconque des revendications 1 à 8 ; ou le composé 3-(3,4-difluorophényl)pyrrolidine ;
l'un quelconque de ses stéréo-isomères ou tout mélange de ses stéréo-isomères, ou un N-oxyde de celui-ci ou un sel pharmaceutiquement acceptable de celui-ci, pour une utilisation dans le traitement, la prévention ou l'atténuation d'une maladie ou d'un trouble ou d'un état d'un mammifère, incluant un être humain, laquelle maladie, lequel trouble ou état réagit à une modulation de la fonction dopaminergique dans le système nerveux central.
